# EUROPEAN PATENT APPLICATION

(11) **EP 3 485 899 A1**
(43) Date of publication of application: **22.05.2019**
(21) Application number: 17904349.2
(22) Date of filing: 25.09.2017
(51) Int. Cl.: A61K 38/19, A61K 31/25, A61K 47/36, A61K 31/721, A61K 47/12, A61K 9/08, A61K 9/10, A61P 31/12, A61P 31/04, A61Q 11/00, A61F 13/15, A41B 15/00, A61L 15/44

(54) **THERMOSTABLE COMPOSITION WITH ANTIVIRAL AND ANTIBACTERIAL ACTIVITY AND USE THEREOF**

(71) Applicant: Chumburidze, Georgy Georgievich, Moscow 125008 (RU)
(72) Inventor: Chumburidze, Georgy Georgievich, Moscow 125008 (RU)
(74) Representative: Bucher, Ralf Christian
(86) International application number: PCT/RU2017/000704
(87) International publication number: WO 2019/059804

(57) **Abstract**

A series of inventions refers to biotechnology, pharmaceutics, medicine, cosmetology, veterinary and can be used for prevention and treatment of bacterial and viral diseases in humans and animals, and also relates to the production of a wide range of sanitary and medicinal products. A thermostable composition with antiviral and antimicrobial activity and its use as a biologically active component for the production of sanitary and hygienic products as well as for protection from bacterial and viral infections has been claimed. In particular, the thermostable composition is a lyophilized powder containing the following components per 1 g of the composition:
recombinant interferon gamma human with the activity from 500,000 to 2,000,000 IU / g 0.03 to 0.12 mg
lysostaphin with the activity from 50 to 200 U / g 0.17 to 0.68 mg
methoxy polyethylene glycol 30 - 150 mg
sodium alginate 10 - 25 mg
sodium dihydrophosphate dihydrate 120 - 250 mg
citric acid 35-110 mg
dextran q.s.

The proposed thermostable composition can be used as a component of oral cavity care products, toothpastes, sanitary-hygienic products, a clothes conditioner and condom lubricant and in a number of medicinal products and drugs.

## Description

### TECHNICAL FIELD

A series of inventions refers to biotechnology, medicine, cosmetology, veterinary and can be applied for prevention and treatment of bacterial and viral diseases in humans and animals, and also can be applied in the production of a wide range of sanitary and hygienic products and medicines.

### THE PRIOR ART

Exposure to various environmental microorganisms and viruses may cause infectious diseases. It is known that sanitary and hygienic measures can significantly reduce the population of microorganisms, including pathogens.

Staphylococcal infections are common anthropozoonotic bacterial infectious diseases with various mechanisms of pathogen transmission. These infections are characterized by the development of purulent inflammation in the infection foci, intoxication and frequent generalization of the pathological process followed by sepsis development. Human skin is inhabited by 14 *Staphylococcus* species. Three of them, including Staphylococcus aureus, Staphylococcus epidermidis and Staphylococcus saprophyticus, are significant in human pathology. Staphylococcus aureus, a persistent highly virulent causative agent easily acquiring resistance to antimicrobial drugs, is the most pathogenic microorganism in humans. Less virulent coagulase-negative staphylococci, Staphylococcus epidermidis in particular, exhibit tropism to foreign substances (endoproteases) and increasingly become hospital infection causative agents, especially in patients with low level of immunity. Staphylococcus saprophyticus, the other coagulase-negative staphylococcus, is a common causative agent of urinary tract infections. Staphylococcus aureus is also an obligate parasite of cattle, horses, pigs, dogs, monkeys and occasionally birds. There are cases of infection of milk from cows with staphylococcal mastitis and subsequent food infection outbreaks in humans.

In the process of vital activity, staphylococcus produces various enzymes and toxins that have a harmful effect on the human body. For example, exfoliatin, a staphylococcal toxin is capable of damaging skin cells, enterotoxin causes food infection and leukocidin destroys leukocytes. For this reason, symptoms of Staphylococcus aureus caused diseases differ significantly and depend on the localization of the infection foci, the presence or absence of concomitant diseases in patients, immune status and on the extent of exposure to environmental factors.

Staphylococcal infections are ubiquitous. The disease emerges throughout a year, both sporadic cases and epidemic outbreaks are registered. Susceptibility to staphylococcal infections is low, and due to constant risk of transmission, up to 40% of adults produce antibodies against *Staphylococcus* and staphylococcal toxins. High-risk groups include newborns and infants under one year as well as immune-compromised patients (HIV-positive persons, drug addicts, patients with diabetes, etc.).

Prophylactic measures and use of hygienic products are aimed to prevent domestic and workplace, food and nosocomial staphylococcal infections. Products, which are used for this purpose should not contain antibiotics.

Antibiotic resistance has been first discovered in *Staphylococcus aureus.* Resistance to methicillin coupled with resistance to other beta-lactam antibiotics has given the name of multidrug-resistant staphylococci MRSA (methicillin-resistant Staphylococcus aureus).

Usually MRSA, in addition to resistance to beta-lactam antibiotics, also exhibit resistance to many other classes of antibiotics, including aminoglycosides, macrolides, fluoroquinolones, clindamycin, co-trimoxazole, chloramphenicol (Infections and Antimicrobial Therapy, v. 7, No. 4, 2005).

Design of new alternative drugs that have antimicrobial activity with a minimal risk of resistant strain emergence that ensure the restoration and normalization of biocenotic interactions in human body and possibility of their industrial application is one of the tasks of science and industry.

Therefore, currently it is relevant to use substances that do not have antibiotic activity. Lysostaphin is a potential option in treatment and prevention of infections. It has a high degree of antistaphylococcal bacteriolytic activity. Lysostaphin belongs to antimicrobial peptides and proteins known as bacteriocins. Bacteriocins have bactericidal activity against other bacteria and provide a self-protection mechanism that helps microorganisms to survive in their natural habitat. Lysostaphin has the ability to provide proteolytic effect on glycine-containing peptidoglycan staphylococcus cell wall bridges. Many studies have demonstrated that this protein can be used for prevention or treatment of bacterial staphylococcal infectious diseases, in particular in combination with other antibacterial agents. Lysostaphin is stored in the form of frozen solution. It is active against all antibiotic-resistant strains of pathogenic staphylococci, including MRSA-strains and is more efficient than other anti-staphylococcal biological preparations, e.g. bacteriophages, bacteriocins of another group and phage endolysines. The properties of recombinant lysostaphin do not practically differ from those of its active natural form.

In addition, protection from viral infections is also the current problem. Viruses are the main category of pathogens. Viral infections are among the main causes of morbidity in humans. Viruses demonstrate a wide diversity of structures and life cycles.

Viruses are obligate intracellular parasites. They have surface proteins that are cable of binding to specific receptor proteins on the surface of host cells. Overcoming species barriers, viruses can transfer individual genes or gene groups from one cell to another. Unlike local pathogenesis in bacteria, viral infections are characterized mainly by their systemic spread to the entire body, infecting the cells of most organs.

Acute respiratory viral infection (A.R.V.I.) is the most common group of viral diseases. Influenza plays a leading role in the structure of A.R.V.I, diseases. About 200 different RNA-containing viruses cause A.R.V.I. providing serious difficulties for prevention and treatment of the diseases. The other challenges include treatment of herpes, papilloma and other viral infections. Parenteral viral hepatitis is one of the most critical problems of infectology. Interferon preparations are important for emergency prevention of various viral infections.

Unlike the natural protein, recombinant gamma interferon human consists of 144 amino acid residues and lacks the first three amino acids Cys-Tyr-Cys replaced by Met, which initiates protein synthesis in bacteria. IFN-gamma monomer has a molecular weight of 16.9 kDa. Biologically active form of IFN-gamma is a dimer. Specific antiviral activity in human fibroblasts, VERO monkey cells or MDBK bull cells infected with vesicular stomatitis virus or encephalomyocarditis virus is about 2x10⁷ IU per 1 mg of the protein. The substance of interferon gamma is stored at minus 20 ° C and remains thermostable only for six months. Also, recombinant gamma interferon human obtained from Escherichia coli strain containing recombinant plasmid DNA pGIF315 is known from the prior art as well as a technology for its isolation and purification (patent RU 2214832, published on October 27, 2003). This recombinant gamma interferon human is manufactured by Scientific Production Plant PHARMACLON, Ltd. (Russia) under the trade name of Ingaron®.

It is known that pegylation (conjugation with PEG) is used to increase the prolonged action of proteins. Pegylation is a process in which polyethylene glycol chains are covalently bound to polypeptides, that allows improving clinical parameters (pharmacokinetics, pharmacodynamics and immunogenicity). The properties of polypeptide properties might be changed, but its activity remains unchangeable as polyethylene glycol molecule protects polypeptide from exposure to external factors.

At the same time, pegylation has certain shortcomings. Chemical synthesis during pegylation leads to polydisperse composition of the product. Molecules with different molecular weights are obtained and therefore may have different properties. For example, molecules with a large number of monomers may be immunogenic and may be accumulated in liver. Loss of specific activity of the product due to impeding of active groups and conformation changes is also one of the most significant shortcomings of pegylation.

Interferon gamma has the lowest thermal stability of all known interferons. This low thermal stability of interferon gamma makes it difficult to use as an active ingredient for the production of various products.

Thermostable variants of interferon gamma human are known from the prior art (patent US 6.046.034, published on April 4, 2000; patent FR 2899589, published on October 12, 2007). These patents disclose genetically engineered thermostable gamma interferons that are obtained by substitution of amino acids in the native structure. However, as a result, there are mutants (analogues) of interferon gamma, whose effect on the human body has not been studied and their commercial applicability still is not clear.

A thermostable lyophilized composition containing a drug and a lipid component consisting of one or more non-ionic surfactants (surfactant) is known from the prior art, and the drug can contain a therapeutic protein comprising an antibody (patent application RU 2014133089, published on March 20, 2016).

Stable pharmaceutical formulations that comprise interferon gamma and alpha in synergistic proportions are also known from the prior art (patent RU 2403057, published on November, 10, 2010).

In addition, a therapeutic antiviral agent containing alpha, beta or gamma recombinant interferon is known. The agent additionally contains a stabilizer with biological, physicochemical properties and / or resistance to microbial contamination and a consistence-forming base (patent RU 2150291, published on 06.10.2000).

Also a method for treating staphylococcal diseases using relatively high doses of lysostaphin of at least 50 mg and preferably 100 mg of lysostaphin per kilogram of body weight is described (US Pat. No. 6,028,051, published on Feb. 22, 2000).

Based on the sum of essential characteristics, an antiviral product consisting of freeze-dried collagen and interferon is the most advantageous technical solution to the claimed thermostable composition (patent for invention RU 2204410, published on May 20, 2003). The drug is effective in treating purulent wounds, but it might be difficult to apply in case of dry wounds and eye mucosa.

The above-mentioned compositions are effective in treating only one type of infection, either viral or bacterial.

A number of compositions and methods of their use for sanitary and hygienic purposes are known from the prior art.

Consumers use a variety of personal and household hygienic products because cleaning the skin and other living and non-living surfaces to reduce microbial populations is the first protective solution to eliminate pathogens from these surfaces and thus to reduce the risk of infection.

These products often include components that destroy or inhibit the growth of unwanted organisms, such as bacteria and viruses. They are used, for example, for skin care, as personal cleansers, for mouth care and can also be used as antimicrobial agents for solid surfaces.

Contamination of the treated objects with residues of toxic substances that require additional measures for their removal is a negative aspect in the use of chemical agents. At the same time, these methods are not environmentally friendly, since they involve the use of chemical agents slowly degrading in the environment. In addition after being used for their intended purpose, many drugs pass into the environment by different ways and contaminate it.

In this regard, it is important to develop tools that contain toxicologically safe and environmentally friendly substances as active ingredients.

For prevention and treatment of periodontosis there is known a gel containing metronidazole benzoate, chlorhexidine gluconate as an active substances, carbomer and hydroxypropyl methyl cellulose as gelling agents, menthol flavor, sweetener, disodium EDTA as an auxiliary component and a filler. The gel can further comprise active substances selected from the group comprising alpha, beta, gamma recombinant interferon, lysine, proline and choline salicylate (international application WO 2014/014389, published on January 23, 2014)
Also, a composite spray is described in the patent for invention CN 103705912, published on 09/04/2014. The spray contains lysostaphin, lysozyme, tea polyphenols, olive oil, vitamin C, lecithin, xanthan gum and purified water.

The most advantageous technical solution for the claimed oral rinsing product is a product for oral cavity care based on lysostatin (patent for invention CN 1360886, published on December 28, 2000). It is capable of treating oral cavity diseases caused by staphylococci, hemolytic streptococcus and anaerobic bacteria.

The known oral rinsing formulations exhibit anti-inflammatory and antimicrobial properties, but the effectiveness of many of them is reduced after a long-term application due to adaptation of the microbial population.

Pharmaceutical compositions for the prevention and treatment of periodontal diseases in the form of pastes, elixirs, rinsing products, ointments, emulsions and gels (patent RU 2228168, published on May 10, 2004; patent for invention RU 2201206, published on 27.03.2003; 2190995, published on October 20, 2002, patent for invention RU 2286764, published on November 10, 2006) are widely known. In the treatment of periodontal diseases, three main etiological directions are used, including antibacterial, anti-inflammatory, regenerating efects; they result in elimination of the infection nidus, resolution of inflammation in the lesions, improvement of the trophic and recovery processes.

Various toothpastes used for the prevention of periodontal inflammatory diseases containing anti-inflammatory and antimicrobial components are known from the prior art, for example medicinal herbal extracts, vitamins, a mixture of lysates (Silca Herbal plus® curative and prophylactic toothpaste (Germany), application for invention RU 2012101121, published on July 20, 2013). The described toothpastes do not provide antiviral effect during application. The disadvantage of these formulations is a low antibacterial effect due to the low bactericidal activity of the components.

The most advantageous technical solution to the toothpaste is an oral cavity care kit in the form of a toothpaste. The toothpaste contains an oral cavity care composition comprising a component containing at least one Eₕ increasing compound and a pharmaceutically acceptable carrier and a component comprising at least one zinc compound, cetyl pyridinium chloride (CPC), and a pharmaceutically acceptable carrier (patent RU 2581915, published on August 5, 2011). In one option, the mouth care formulation additionally contains an antiviral agent. Suitable antiviral agents for use in mouth care formulations include interferon of type III, type II and type I.

However, the known agents provide either antibacterial or antiviral effect.

Various compositions can also be used for manufacturing different products, including sanitary and hygienic products, cosmetics, etc.

Wiping spray is used for cleaning and disinfection of living or non-living surfaces. Examples of such products are provided below.

From the prior art, an unwoven fabric that is used in products for collection of human body excretions in particular disposable diapers treated with linear polymeric biguanide for prevention of bacterial activity are known (EP 0174128, published on March 12, 1986).

A skin care formulation, a paper towel containing lysostaphin and Chinese herbal extracts as well as application instruction are described in patent for invention CN 103861092, published on June 18, 2014.

There are known antibacterial dressings Neotulle manufactured by Fisons Company (Germany) and Sofratulle produced by A.Roussel Company (Germany) which represent sterile wipes on a woven network fabric base impregnated with neomycin or framicetin (a synonym of neomycin) based on Vaseline or white solid paraffin in combination with lanolin (Martindal the Extra Pharmacopoeia, London, 1978, v. 27, p.1159).

However, these wipes contain antibiotic neomycin that has a high toxicity and nephrotoxicity and is not active against infections caused by resistant Gram-negative microorganisms.

A wet paper or fabric wipes that are impregnated with a formulation containing bacteriocin, chelating agent, stabilizer, surfactant, salt and alcohol as a drying agent is the most advantageous technical solution to a sanitary skin treatment product (international application WO 96/39842, published on December 19, 1996). The method of disinfecting, drying and / or cleaning the surface includes wiping the surface with a sanitary wipes having a disinfecting effect.

The disadvantage of the known solution is the use of alcohol that cause drying and pilling of skin.

Based on the sum of essential features, a wipe that is used for disinfecting hands, surfaces, injection site or skin of the elbow bend in donors is the most advantageous solution of the claimed sanitary hygienic wet product (patent RU 2253481, published 10.06.2005). The wipe is made of absorbent material and impregnated with a liquid disinfectant. Such wipes have no antiviral activity.

Based on the sum of essential features, a disposable absorbent product, hygienic tampon, sanitary wipe, daily liner is the most advantageous technical solution of the claimed disposable absorbent product (application for invention RU 2010141844, published on 20.04.2012). However, these products do not possess antibacterial and antiviral activity.

There is known a hand care product prepared with a certain ratio of non-ionic surfactants, including zwitterionic surfactants, anionic surfactant, lanolin, 1,3-propanediol, glycerol, citric acid, allantoin, bacteriostatic agent, lysostaphin and deionized water (patent CN 102846496, published September on 18, 2012). The product inhibits the growth of harmful bacteria only and does not possess antiviral activity.

A medicinal gel with recombinant interferon and vitamin E is known. The gel contains methionine, citric acid, benzoic acid, human serum albumin, ethyl alcohol, glycerin (patent RU 2184564, published on July 10, 2002).

Based on the sum of essential features, a product exhibiting immunomodulatory, antimicrobial, antioxidant and regenerative effect is the most advantageous technical solution of the claimed skin care products. The product contains recombinant interferon, lysozyme, emoxypin and at least one auxiliary substance: either hydrophilic base, acetate buffer or a preservative (patent for invention RU 2255760, published on July 10, 2005).

However, these compositions, like those listed above, have either antibacterial or antiviral effects.

Chemical methods based on the use of disinfecting chemicals (formaldehyde, glutaraldehyde, peracetic acid, phenolic, cresol, iodomorphic solutions, etc.) are most frequently used (patent for invention RU 2148415, published on May 10,2000; invention RU 2163146, published on February 20, 2001) to disinfect a wide range of objects.

Antimicrobial compositions containing a disinfecting alcohol, an organic acid and water are described in patent RU 2366460, published on January 20, 2009, and the composition with pH of about 5 or lower exhibits disinfecting effect against bacteria and /or is capable of synergistically inactivate or destroy viruses.

A preparation containing hydrogen peroxide, a quaternary ammonium compound, an organic acid or a mixture of two or more organic acids and carbamide in certain proportions is known. A method for the preparation of a product with the above components is also provided. The resulting preparation has high antimicrobial activity and thermal stability (patent for invention RU 2180572, published on 20.03.2002). This product is not friendly to the environment.

A method for disinfection of premises and equipment, preservation and water decontamination using a chlorhexidine-based agent is described in international application WO 2008/108680, published on September 12, 2008. Chlorhexidine (1.1-hexamethylene bis-parahlorphenyl-bis-guanidine) as an antiseptic agent is manufactured by many companies. This product is not friendly to the environment.

A disinfecting gel containing lysostaphin, bactericidal peptide, chlorhexidine, sorbitol, sodium dihydrogenphosphate, sodium hydrogen phosphate and a thickener is the most advantageous technical solution to the claimed sanitary-hygienic products (patent of invention CN104524557, published on April 22, 2015).

This product is not friendly to the environment.

It is known that lysostaphin is used in compositions for treatment of clothes (international application WO 2002/066591, published on August 29, 2002, patent for invention CN 1364866, published on January 11, 2001).

A composition containing surfactant, enzyme and lysostaphin is the most advantageous technical solution to the claimed clothes conditioner (application for invention US 2002 / 178,509, published February 17, 2001).

However, the known products do not have antiviral activity.

Outside and inside lubrication of condom with a disinfecting agent is known to prevent sexual transmission of HIV (EP 0427997, published May 22, 1991).

A condom lubrication agent in the form of gel or ointment containing disinfectant, a substrate and menfegol as an anti-inflammatory agent is disclosed in EP 0343631, published on November 29, 1989.

Based on the sum of essential features, a condom lubricant comprising antiviral preparation and pharmaceutically acceptable target additives is the most advantageous technical solution to the claimed product (patent RU 2187329, published December 18, 2000). The lubricant contains alpha, beta or gamma INF as an antiviral component in an amount of 1000-10000000 IU per 1 g of the product.

Along with sanitary-hygienic products, medicines exhibiting antimicrobial, antiviral effect are widely known.

Medicinal product that exhibits immunomodulating, antimicrobial, antioxidant and regenerating effect known in various mouth, parenteral and topical dosage forms (tablets, spray, solutions, gels), for administration via mucous membranes (suppositories). The product is based on natural and recombinant interferon α, β, γ, lysozyme and emoxipin (patent for invention RU 2255760, published on July 10, 2005). Use of an unstable component such as gelatin and chlorhexidine preservative that may reduce the stability of interferon in the hydrophilic base of suppositories is the disadvantage of the present medicine. Tablet dosage forms of this drug contain large amount of lactose (0.3 g). Lactose is known to cause digestive disorders in many patients due to reduced activity or lack of the corresponding enzyme, lactase. Stability of interferon may be compromised in the process of tablet production due to temperature increase during compression.

Stabilized drug Viferon-forte is based on human interferon recombinant alpha and / or beta and / or gamma with an antioxidant complex including tocopherol acetate, ascorbic acid and their derivatives, and also containing auxiliary substances such as stabilizers, buffer solutions, acids, plasticizers, emulsifiers, preservatives and a substrate. The drug is known for treatment of infectious inflammatory diseases with high degree of bioavailability (patent for invention RU 2381812, published February 20, 2010). This drug possesses antibacterial, antiviral properties and is used in treatment of infectious and inflammatory diseases of urogenital tract, viral hepatitis and other infectious pathologies. In addition, various fats and oils requiring special stabilization with a large variety of substances are proposed as a base of the product.

A preparation of genetically engineered gamma-interferon comprising interferon, stabilizing additives, biologically inert filler, amino acid or salt thereof and saline buffer system in a specified ratio of components is known (patent RU 2077336, published on 10.06 1997).

There is a drug for the treatment of viral, chlamydial and bacterial infections based on interferon. It is a single-unit dosage form comprising a mixture of interferon and alpha-tocopherol acetate as an antioxidant substance. The drug may represent a rectal capsule or suppository and administered per rectum (patent for invention RU 2057544, published on April 10, 1996).

A medicinal preparation for the treatment of viral infections is known. It is made in a single dosage form of a mixture of interferon and a substance containing at least 2.2% polyunsaturated fatty acids. The preparation may represent a rectal capsule or suppository and administered per rectum (patent RU 2097061, published on 27.11.1997).

Based on the sum of essential characteristics, an antiviral drug comprising human interferon and a synergist is the most advantageous technical solution to the claimed medicinal product (patent RU 2073522, published on February 20, 1997). It contains human immunoglobulins as synergistic agents, recombinant human alpha, beta, or gamma interferon as human interferon, a mixture of IgA, IgM and IgG as human immunoglobulins and pharmaceutically acceptable target additives. Technical result of the invention is the design of a prolonged action preparation of recombinant interferon that provides good penetration and has antiviral, antibacterial, anti-inflammatory, detoxicant, local anesthetic, and regenerating effect. The antiviral agent is prepared either in the form of ointment of various consistencies or in the form of suppository.

A disadvantage of the formulations described above is that almost all of them contain antibiotics, lysozyme and chlorine-containing compounds. Apparently, this is due to the fact, that the known solutions and suspensions containing interferon do not have a long shelf life because of their rapid inactivation during storage.

Based on the sum of essential characteristics, a medical product in the form of bandage for treatment of wounds and burns is the most advantageous technical solution to the claimed product (international application WO 2014/062080, published on April 24, 2014). The bandage comprises an absorbent material representing a nonwoven perforated fabric impregnated with a gel mixture of boric acid, hydroxypropylmethylcellulose, lidocaine hydrochloride and purified water with the following ratio of the components in mass %: boric acid 0.001-20.0, hydroxypropylmethylcellulose 0.001-10.0, lidocaine hydrochloride 0.001-10.0, the rest amount being purified water. The product further contains betamethasone in an amount of 0.0001-1.0 or a recombinant interferon alpha, beta or gamma in amount of 100-1000000 IU or heparin in amount of 10-100000 IU. However, the product described does not have antiviral activity.

State of the art analysis has shown that due to the fundamental differences between bacteria and viruses it is difficult to prepare an antimicrobial composition effective against both bacteria and viruses. Only compositions which comprise either lysostaphin or interferon gamma are known. In addition, there are only a few commercially available products containing antibacterial and antiviral substances and possessing biocidal and protective effect. Examples of combined use of lysostaphin and interferon gamma are not known.

Perhaps this is due to the fact, that the optimal conditions for manifestation of biological activity of interferon gamma and lysostaphin are not identical. For example, human recombinant interferon gamma is stable and is stored at pH 6.0-6.5, whereas lysostaphin works better in low alkaline conditions (pH 7.5-7.8). Under these conditions, there is a risk of aggregation of interferon gamma molecules and, as a consequence, a decrease of its activity. That is why preparations based on interferon gamma (for example, "Inharon") with pH 7.5-8.0, have a shelf life of 2 years at a temperature of plus 2 to 8 °C. The fact that different proteins behave differently is related to the amino acid composition (primary structure) of them and physicochemical properties.

### DESCRIPTION OF THE SERIES OF INVENTIONS

Obviously, there is a need to design environmentally friendly, harmless and effective compositions with a broad spectrum of action against viral and bacterial infections that would retain stable biological activity during storage and that would have broad industrial applicability.

The invention proposed is intended to resolve the abovementioned problems.

Design of a highly active composition of bioavailable substances that would retain their thermal stability under standard storage conditions for prolonged time and would exhibit antiviral and antimicrobial effect and that could be used for manufacturing of various products, in particular medicinal products, without loss of the activity is the objective of the proposed invention. Another purpose is to extend the range of hygienic and medicinal products that lack toxicity, allergic capacity and other side effects.

Results achieved by technical solution of the abovementioned technical problems, include the following:
- extension of storage temperature range for recombinant interferon gamma human and lysostaphin;
- provision of a long-term (not less than 5 years) stabilizing effect under standard storage conditions by maintaining and stabilizing the biological properties of two different proteins, recombinant interferon gamma human and lysostaphin due to the optimal formulation and component ratios;
- design of a long-term (not less than 5 years under standard storage conditions) thermostable, bioavailable, ecologically pure, non-toxic, hypoallergic composition with high antiviral and antibacterial activity, high biocidal effect as well as providing good penetration ability and a wide range of application (mucous membranes, skin, surfaces, clothes, etc.) on the base of gamma-IFN human and lysostaphin;
- use of the thermostable composition in various products that provide comprehensive protection of humans against bacterial and viral infections by stabilizing it that allows the claimed products to be stored under standard conditions for a long period of time (not less than 3 years) with no loss of high antiviral and antibacterial activity;
- ensuring the possibility to apply the thermostable composition without losing its properties and activity during a large-scale production of the claimed products, the commercialization being achieved in a short time;
- promotion of the products containing highly active stabilized components;
- providing the possibility of obtaining various end-products with different consistency, organoleptic properties using standard equipment for production of liquid and semi-solid medicinal or cosmetic products;
- formation of uniform thin films on the surface of skin, mucous and non-living objects that allows the claimed composition to retain biological activity against pathogenic microorganisms and viruses for a long time. Sanitary and hygienic products on the base of the claimed composition should have high wettability and adhesive properties to the treated surfaces.

These technical results have been achieved due to the original thermostable composition possessing antiviral and antimicrobial activity and its use for production of sanitary-hygienic products, medicines as well as products for protection from bacterial and viral infections. The claimed series of inventions is united by a single inventive concept.

The abovementioned technical results were achieved by designing a thermostable antiviral and antibacterial active formulation in lyophilized form, 1 g of the formulation containing the following ingredients:
recombinant gamma interferon human
with the activity from 500.000 to 2.000.000 IU / g0.03 to 0.12 mg
lysostaphin with the activity of 50 to 200 U / g 0.17 -0.68 mg
methoxy polyethylene glycol 30-150 mg
sodium alginate 10-25 mg
   sodium dihydrophosphate dihydrate 120-250 mg
citric acid 35-110 mg
dextran q.s.

In this case the thermostable composition preferably comprises a lyophilized powder obtained from a solution with pH 5-7.

Recombinant gamma interferon human constructed from plasmid pGIF315 transformed E. coli strain is preferably used as a human recombinant interferon gamma.

Dextran with molecular weight from 60.000 to 70.000 is preferably used.

Methoxy polyethylene glycol with molecular weight from 2000 to 5000 is preferably used.

Recombinant lysostaphin can be used as lysostaphin.

An oral cavity care product represents an aqueous solution of the thermostable composition in any of the above combinations and the aqueous solution has the activity of recombinant gamma interferon human 200-1000 IU / ml and that of lysostaphin 0.02-0.1 U / ml. The product may further contain sorbitol, sodium chloride and a preservative in one of the combinations and povidone, potassium sorbate and / or potassium lactate, flavoring agent and a preservative in the other. In the last combination, the oral cavity care product is produced in the form of spray.

A toothpaste is also claimed, which contains the thermostable composition in any of its above combinations and suitable fillers. The toothpaste comprises recombinant gamma interferon human and lysostaphin with the activity of 200-1000 IU / g, and 0.02-0.1 U / g, respectively, and the following ratio of components (mass%):
thermostable composition 0.01-0.2
suitable fillers q.s.

In addition, the toothpaste may additionally contain vitamins, and / or antifungal agents or a mixture of them.

A sanitary and hygienic product comprises a fabric base and the thermostable composition in any of its above combinations. The activity of recombinant interferon gamma human and lysostaphin is 5000-20000 IU / cm² and 0.5-2 U / cm², respectively. The ratio of components is as follows, %):
thermostable composition 0.25-4
fabric base q.s.

In this case, the fabric base is impregnated with the thermostable composition.

The sanitary and hygiene product may further contain carboxymethyl cellulose, sodium alginate, trehalose and a preservative, which, before mixing with the thermostable composition, is dissolved in water and the base is moistened with the resulting aqueous solution and then dried to a moisture content of 1-7 wt%. The end product contains the following ration of components (mass%):
thermostable composition 0.25 - 4
carboxymethylcellulose5-40
trehalose 0.05-0.5
sodium alginate 1-5
preservative 0.1-0.5
fabric baseq.s.

The fabric base is preferably composed of cotton, and / or cellulose and / or viscose, and / or synthetic materials or a mixture of them.

The sanitary product may be one of the following: a wipe, cosmetic mask, paper towel, disposable gloves and handkerchiefs.

In this case, the product is packaged in an individual hermetic pack or in a batch of hermetic packs.

Sanitary hygienic wet product is a fabric base impregnated with the thermostable composition in any of its above combinations dissolved in an aqueous solution containing carboxymethylcellulose, sodium alginate and a preservative and dried to a moisture content of 10-20 wt%, the activity of human recombinant interferon gamma and lysostaphin being 4000-16000 IU / cm² and 0.4-1.6 U / cm², respectively. The ratio of components in the end product is as follows (mass%):
thermostable composition 0.2-3.2
carboxymethylcellulose 10-25
sodium alginate 2-10
preservative 0.05-0.15
absorbent material q.s.

In this case, the absorbent material may consist of cotton, and / or viscose, and / or synthetic materials, or mixtures of them.

The sanitary hygienic moist product may further comprise glycerol and / or thioglycol and / or antifungal agents and / or vitamins and / or a dye and / or a flavor or a mixture of them.

A sanitary moistened product may represent wipes, moist toilet paper or hygienic tampons.

In this case, the product is packaged in an individual hermetic pack or in a batch of hermetic packs.

A disposable absorbent product comprises a carrier and a sanitary-hygienic product in any of its abovementioned compositions, with a weight-to-weight ratio of substrate: product (5-10): (1-5).

The disposable absorbent product may preferably be one of the following: a tampon, sanitary wipe and a hygienic liner or a daily liner.

In this case, the product can be placed in an individual hermetic pack or in a batch of hermetic packs.

A skin care product comprising a cosmetically suitable base and the thermostable composition in any of the abovementioned combinations should have the activity of recombinant interferon gamma human and lysostaphin of 500 to 2000 IU / g and 0.05 to 0.2 U / g, respectively, the following ratio of components in the end product being as follows (mass%):
thermostable composition 0.025-0.4
cosmetically acceptable substrate q.s..

The cosmetically suitable substrate may preferably comprise a gel, fat, aqueous solution, powder, talc and AERSIL®.

The skin care product may further comprise spermocite and / or vitamins and / or mineral salts and / or antifungal agents or a mixture of them.

The skin care product may preferably be in one of the following forms: a cream, gel, jelly, powder, lotion, spray or a mask.

The skin care product may be of veterinary use.

The sanitary-hygienic product comprises an acceptable liquid substrate and the thermostable composition in any of its the abovementioned combinations, the activity of recombinant interferon human and lysostaphin being 500 to 100.000 IU / ml and 0.05 to 10 U / ml respectively. In the end product the ratio of the components should be as follows (mass%):
thermostable composition 0.025 - 20
acceptable liquid base q.s.

The sanitary-hygienic product may further contain trehalose, a preservative and PEG 400 and represent a spray.

The hygiene product may further comprise triethanolamine to provide pH from 6.0 to 8.0.

The sanitary-hygienic product may be in the form of gel and further contain carboxymethyl cellulose, sodium alginate and a preservative.

The sanitary-hygienic product may be a product for surface treatment or a product for clothes treatment.

A laundry conditioner comprises an acceptable liquid base and the thermostable composition in any of the abovementioned combinations with the activity of recombinant interferon gamma human and lysostaphin being 2000-10.000 IU / ml and, 0.2-1 U / ml, respectively. The end product should contain the following ratio of components (mass%):
thermostable composition 0.1 - 2
acceptable liquid baseq.s.

A condom lubricant comprises the thermostable composition in any of the abovementioned combinations and pharmaceutically acceptable additives, the activity of recombinant interferon gamma human and lysostaphin being 5 to 50 IU / g and 0.0005 to 0.005 U / g, respectively, with the following component ratio (wt%):
thermostable composition 0.00025 - 0.01
pharmaceutically acceptable
target additivesq.s.

The product may further contain vitamins and / or antifungal agents.

The medicinal product comprises the thermostable composition and pharmaceutically acceptable base, with the activity of recombinant interferon gamma human and lysostaphin being 5000-100000 IU / g and 0.5-10 U / g, respectively, with the following component ratio (wt%):
thermostable composition 0.25 - 20
pharmaceutically acceptable base q.s.

In this case, the medicinal product may be in the form of suppository, spray, ointment, gel or lyophilized powder.

The product may comprise a fabric base and the thermostable composition, the activity of recombinant interferon gamma human and lysostaphin being 5000-20000 IU / cm² and 0.5-2 U / cm², respectively, and the component ratio being as follows (mass%):
thermostable composition 0.25 - 4
fabric base q.s.

The medical product may further contain carboxymethylcellulose, sodium alginate, trehalose, preservative that are dissolved in water and moisten the fabric base that is then dried to a moisture content of 1-7 wt% before mixing with the thermostable composition, the following ratio of the components in the end product being as follows(mass%):
thermostable composition 0.25 - 4
carboxymethyl cellulose 5 - 40
trehalose 0.05-0.5
sodium alginate 1 - 5
preservative 0.1 - 0.5
fabric base q.s..

The product can be obtained by applying the thermostable composition to the fabric base.

In the medical product, the fabric base consists of cotton and / or cellulose and / or viscose and / or synthetic materials or a mixture of them.

The medical product may preferably be in one of the following articles: cosmetic face mask, bandage, disposable gloves or sponge for topical use.

The product may be placed in a sealed individual pack or in a batch of hermetic packs.

As used herein, "lysostaphin" is any lysostaphin, natural or recombinant, providing proteolytic effect on staphylococcus cell wall glycine-containing peptidoglycan bridges. The enzyme (EC 3.4.99.17) represents an extracellular zinc-containing glycyl-glycine endopeptidase. The manifestation of the enzymatic activity of lysostaphin is the destruction of the cell walls of microorganisms of Staphylococcus genus (staphylococcus lysis) by hydrolysing peptidoglycan polyglycine interpeptide bridges found only in the cell wall of staphylococci. Lysostaphin also has elastolytic activity. Lysostaphin is commercially available from several suppliers, for example, Sigma-Aldrich, Inc.

As used herein, "recombinant interferon gamma human " is a protein molecule consisting of 144 amino acid residues binding to IFNGR receptor, which consists of IFNGR1 and IFNGR2 chains. It has antiviral and immunomodulatory properties, activates the work of certain types of leukocytes to protect infection, blocks the formation of viral proteins and the assembly of mature viral particles, causes the death of intracellular microorganisms, destroys virus-infected cells. For implementation of the invention, recombinant gamma interferon human constructed from Escherichia coli strain containing recombinant plasmid DNA pGIF315 (patent RU 2214832, published on October 27, 2003) is used. Recombinant interferon gamma human is commercially available from several suppliers, for example, Sigma-Aldrich, Inc, recombinant gamma interferon human obtained from Escherichia coli strain containing recombinant plasmid DNA pGIF315 is manufactured by Farmaklon, Ltd.

As used herein, "standard conditions" designate standard ambient temperature and pressure, which can also be called normal conditions (NC): pressure 100 kPa, temperature + 25 ° C.

As used herein, «thermostability» is ability of proteins to retain a unique 3D structure of their polypeptide chain and, therefore their activity, in the temperature range from -70 °C to 30 °C.

As used herein, "acceptable" (a suitable substrate, a cosmetically acceptable base, a suitable liquid base, pharmaceutically acceptable additives) designates that the components that make up the formulation are soluble, non-toxic, anionic surfactant free and do not inactivate the proteins used. They do not adversely affect the antiviral and antimicrobial efficacy of the composition and, in particular, do not adversely affect the skin and mucosa, and their use is common in this particular field.

The thermostable composition according to the claimed invention provides effective antiviral and antimicrobial activity for a long time.

When designing the claimed composition consisting of a complex of two proteins, a number of problems related to storage temperature, stability, shelf life and stability, when used as raw material for the production of sanitary-hygienic product, has been faced.

Thus, the main problem associated with protein compositions is to make proteins physically stable. Physical instability does not cause changes of covalent bonds in proteins. It probably involves a change in the structure of proteins of a higher order, for example, a secondary structure. Such changes include denaturation, adsorption on surfaces, aggregation and precipitation.

Usually, protein denaturation is observed under the influence of abnormal temperature, pH, etc. Proteins can be also denatured by cold. In addition, proteins are denatured under the influence of too high or low pH, causing the disappearance of a charge of one of the parameters (A.V. Finkelstein, "Protein Physics", M2002).

It is known that the above types of instability, which can have a strong effect on the industrial viability and efficiency of protein compositions, are difficult to eliminate.

It is known that protein stability can be improved by introducing excipients into the composition that interact with the protein in the solution, making it stable, soluble, and not aggregated. For example, salts of compounds and their ionic forms are commonly used as additives introduced into protein compositions. Such compounds help to eliminate denaturation of proteins, forming non-specific bonds with proteins and increasing thermostability. Salts (e.g., NaCl, KCl), amino acids (e.g., histidine, arginine), decrease the change in secondary protein structures (Tian et al., Int'l J. Pharm. 355, 20 (2007)). Other examples of commonly used additives include polyhydric alcohols, such as glycerol, sugars and surfactants. However, the performed experiments showed that the use of the above substances in the recommended doses failed to stabilize the mixture of recombinant interferon gamma human and lysostaphin.

Protein-based products should often have high concentration to achieve positive effect. The performed experiments have shown that the use of these substances in the recommended doses makes many problems, including manufacturing, stability maintaining, delivery, which is especially characteristic for proteins. And there are also problems associated with protein product opalescence and aggregation. In addition, self-association of two main proteins leads to an increase in the viscosity of the solution, which makes it difficult to obtain high protein concentrations during filtration. Therefore, the limits of the quantitative content of proteins as well as the quantitative and component composition necessary for their stabilization should be determined experimentally. Thus, the upper limit of interferon gamma is 2.000.000 IU / g, for lysostaphin it is 200 units / g. The lower one is also determined experimentally and comprises 500.000 IU / g and 50 U / g for interferon and lysostaphin, respectively.

It should be noted that use of recombinant interferon gamma human obtained from Escherichia coli strain containing recombinant plasmid DNA pGIF315 is a preferable combination of the claimed thermostable composition. This is due to the following reasons.

Recombinant plasmid pGIF315 contains promoters providing high constitutive expression gene encoding gamma-interferon of a size of 144 aminoacids with a molecular weight of 16.9 kD in E. coli cells (patent for invention RU 2214832, published on 27.10.2003). This, other factors being equal, allows the use of ordinary nutrient media that do not require the deficiency of any components or the addition of any inducers, which simplifies the technology and reduces the production cost. The plasmid can be transformed into any strains of E. coli that meets the requirements for producer strains, for example, SG 200-50 or BL 21.

Surprisingly, it has been found that methoxypolyethylene glycol, sodium alginate and dextran in the claimed amount can reduce or prevent the effect of both external factors and negative interaction / reaction between biologically active proteins contained in the thermostable composition during the formation of a stable complex.

As shown by the studies performed, use of the thermostable composition in a lyophilized form is the most optimal due to easy manufacture as well as convenience in transportation and storage, because there is no need to follow temperature regime. The obtained dry preparation is a white lyophilized powder that is well dissolved in water and saline for injection to form a clear, colorless or slightly yellowish solution. The obtained product possesses a controlled antiviral and antibacterial activity.

Stable antimicrobial and antiviral effect is ensured by stabilization of recombinant interferon gamma human and lysostaphin in the composition that is provided by optimal formulation and ratios of components. This has allowed for extending the shelf life to 5 years without loss of biological activity. Apparently, in the obtained composition the active substances are protected from external factors because they form a highly active biological complex supposedly enclosed in a nanocapsule-like micelle.

In these compositions, lysostaphin provides direct activity against staphylococci, while recombinant human interferon gamma exhibits antiviral activity when virus enters the body via skin or mucous.

Viral infection activates the expression of interferon-encoding genes. As a result, antiviral activity, blockade of viral protein synthesis and modulation of the immune response take place.

The fact that viruses multiply only in living cells is of critical importance. Since the cell is protected by the cell membrane, it is difficult for the virus to enter the cell, because its dimensions and membrane thickness are almost identical. Viruses have specificity with respect to the surface of certain cells. For infection of the host cell, virus specifically interacts with the cell surface and after a certain interaction time, the viral and host cell membranes are fused. The result of this long-term contact is the penetration of the viral particle or its nucleic acid into the cell. Therefore, in order to destroy the virus that is in contact with human tissues, it is necessary to provide them with a permanent antiviral activity. In this case, the most important role is played by the time factor: the earlier the human recombinant interferon gamma enters the cell, the more effectively the immune system suppresses the virus.

The role of recombinant interferon gamma human in the processes of inhibiting the penetration of virus into cells and synthesis of its proteins has been studied in various models of viral infections. It has also been shown that the antiviral activity of interferon is higher with preventive use. (Kiselev O.V. "Interferon Gamma: A New Cytokine in Clinical Practice", Moscow, 2007).

When designing the thermostable composition, the major criterion was to preserve recombinant interferon gamma human and lysostaphin in their active forms for a long period of time to ensure the shelf life of sanitary-hygienic products for 3 years.

Stable state of the proposed thermostable composition allows for using it in various products that provide a comprehensive protection of human body from penetration of bacterial and viral infections.

On the basis of the thermostable composition, the present invention provides a series of protection products comprising formulations for the production of sanitary and hygienic products, skin care and mouth care products, condom lubricants, clothes conditioner. Some products (or their forms) that contain the thermostable composition can be further stabilized by the introduction of additional components.

Thus, the mouth care product in one form may further contain sorbitol, sodium chloride and a preservative in the amount of 0.15-0.75 (wt%), 0.003-0.015 (wt%) and 0.03-0.0, 15 (wt%), respectively. Alternatively, it may further comprise povidone, potassium sorbate and / or potassium lactate, flavoring agent and a preservative in the amount of 0.006-0.018 (wt%), 0.0003-0.006 (wt%), 0.0001-0.003 (wt%) and 0.03-0.05 (wt%), respectively, and the product may be in the form of spray.

The claimed sanitary-hygienic means and products, when used for skin or mucous care, have a directly pronounced biocidal effect due to strengthening the primary immune barrier.

When designing the sanitary-hygienic products in the form of spray, it has been taken into account that microbes and viruses that have to be inactivated are suspended as microdroplets of moisture and dust particles. For this purpose, the indoor air should be treated with aerosol consisting of microdrops, which are connected with particles contaminated with microbes and viruses thus providing a disinfecting effect.

Sanitary-hygienic products in the form of gel provide prolonged biocidal effect due to the formation of thin protective biodegradable film. In this case, the gel can be used for intimate hygiene before sexual intercourse.

In one version the sanitary-hygienic agent may be a spray and further contain trehalose, a preservative and PEG 400 in the amount of 2-5 (wt%), 0.04 to 1.5 (wt%) and 0.3 to 1.5 (wt%), respectively.

In the other version, it may further contain carboxymethylcellulose, sodium alginate, a preservative in the amount of 10 (wt%), 2 (wt%) and 0.05 (wt%), respectively, and represents a gel.

The effect of various components and physical and chemical factors on activity and thermal stability during storage was assessed using equipment, chemicals, and standard methods known from the prior art. Components of the thermostable composition formulations are known from the prior art and are commercially available.

The water used in the present invention should be of the required degree of purification: purified or distilled, or for injection, or deionized.

Polyethylene glycol ethers PEG40, PEG 4000, PEG 400 and nonionic detergent Tween 80 were used to improve wettability and uniformity of distribution of the products on the surface to be treated, to ensure a high degree of dispersion and thermal stability as well as to avoid the conglomeration of product components that promotes the manifestation of biological effect of the products.

Salts of organic acids, such as benzoic, sorbic or lactic taken in any combination may be used as preservatives to ensure the required microbiological purity.

Various substrates are used to design a skin care product. Thickening agents, for example carboxymethylcellulose, povidone, sodium alginate, pectin are applied to make a cosmetically acceptable gel base. Thickeners are generally present in the amount from 4 to 8% of the total weight of the composition.

The cream base can be both hydrophilic and hydrophobic. It should be noted that the thermostable composition does not cause irritation of skin, nor does it cause corrosion of non-living object surfaces.

Optional components include flavors that are routinely used in compositions in amounts from approximately 0.001 to 5% wt. of the total weight of compositions. The sweeteners are selected from the group comprising mannitol, sorbitol, sucrose, dextrose and glucose.

pH of the compositions designed in the present invention can be adjusted by using buffer components, such as sodium phosphate and citric acid. Triethanolamine was used to adjust pH. Optimal pH was established experimentally. At pH less than 5.0 and more than 7.0 recombinant interferon gamma human and lysostaphin lose their activity and become less stable during storage.

The conducted studies on thermal stability of various proposed compositions (using various combinations of auxiliary substances) under different temperature regimes have shown that the compositions retain their properties during storage at ambient temperatures up to + 25 ° C for 3 years and at +30 ° C for at least 2 years. The proposed thermostable composition can be stored in a wide temperature range from 0 to +30 ° C and has a wide spectrum of antimicrobial and antiviral activity that has allowed for diversification of the products and articles for various purposes and with different ways of application, including use for humans or animals or for surface treatment, external application and application in the form of spray.

The thermostable composition has a wide range of application because of the components it comprises. Human recombinant interferon gamma and lysostaphin are natural factors that protect human body from infections. This ensures bioavailability, hypoallergenic properties, environmental safety and the lack of side effects.

The compositions designed in the proposed invention have a wide range of application, including mouth rinsing liquids, body sprays, antiseptics, wipes, disinfectants, toothpastes, creams and the like for personal care. The thermostable composition can also be used in the composition of sanitary-hygienic products for surface treatment.

The thermostable composition designed in the proposed invention is effective in the manufacture of sanitary and hygienic products having disinfecting properties. It can be used in the household (e.g. for disinfecting solid surfaces such as floors, table tops, home ware and soft materials such as clothes), in personal and intimate hygiene (for example, creams, lotions, gels and sanitary wipes) as well as in industry and hospitals because it effectively and quickly disinfect surfaces that are infected or contaminated with bacteria and viruses.

The thermostable composition can also be included into a fabric base used as a sanitary product with biocidal effect, for wiping skin, for example.

This composition can be used for the production of medical products (bandages, gloves, masks). Use of the thermostable composition for manufacturing medicines in the form of spray, gel, ointment, suppository, lyophilized powder for the preparation of solutions for intranasal use is of particular benefit. These products can be used for treatment of humans and animals.

Designed in the proposed invention, the thermostable composition and the products containing it provide advantages because they are not toxic, hypoallergenic and have no side effects (irritation, xerposis).

Thus, the proposed series of inventions is united by a single inventive concept aimed at designing a thermostable composition with antiviral and antibacterial activity as well as at the use of it in the production of a number of products and articles of sanitary, hygienic, medical, cosmetic application in the form of medicines, toothpaste, mouth care products, laundry conditioners and condom lubricants containing the proposed thermostable composition with high antiviral and antibacterial activity.

Two series of the thermostable composition prepared according to Examples 1 and 2 demonstrated below were used to test antiviral and antibacterial activity at different storage temperatures. After lyophilization, the compositions were subdivided into three equal groups and stored under different temperature conditions: under standard conditions (+ 25 ° C) C), at + 30 ° C and at + 35 ° C.

During the first six months the thermostable compositions were sampled for testing antiviral and antibacterial activity weekly. Subsequently, analytical samples were taken every three months.

An in vitro test system on the base of Vero cells was used for antiviral activity testing. Vesicular stomatitis virus (VSV) was used as a test object. VSV was taken at a concentration of 10 TCD₅₀. Samples were prepared by dissolving 200 mg of the thermostable composition of the first and second series, respectively, in 1 ml of water for injection. The results are shown in Table 1.

A test (reference) strain of staphylococcus was used to test the antibacterial activity of the thermostable composition. The test strain cell suspension was adjusted to a concentration of 1 × 10⁵ cells / ml. Then tenfold dilution was made to obtain a concentration of 1x102 cells / ml. 1 g of the thermostable composition of each series was dissolved in 10 ml of water for injection. Then one ml of the resulting solution was added to one ml of the previously obtained suspension at various dilutions. The solution was incubated for 10 minutes. The results were recorded spectrophotometrically at a wavelength of 600 nm. The results are presented in Table 2.

Specially prepared lyophilized mixtures of recombinant interferon gamma human in a medium of phosphate buffer saline with an activity of 500,000 IU (reference sample No. 1); lysostaphin in a medium of phosphate buffer saline with an activity of 50 U (reference sample No. 2); a mixture of recombinant interferon gamma human (500,000 IU) and lysostaphin (50 U) with phosphate buffer saline with no additives (reference sample No. 3) were used as reference samples.

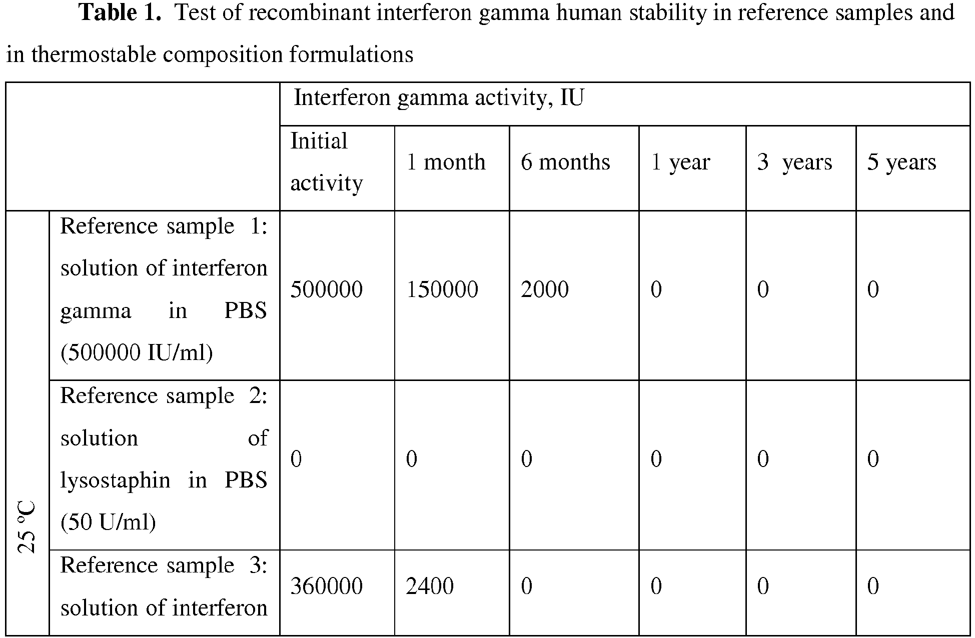

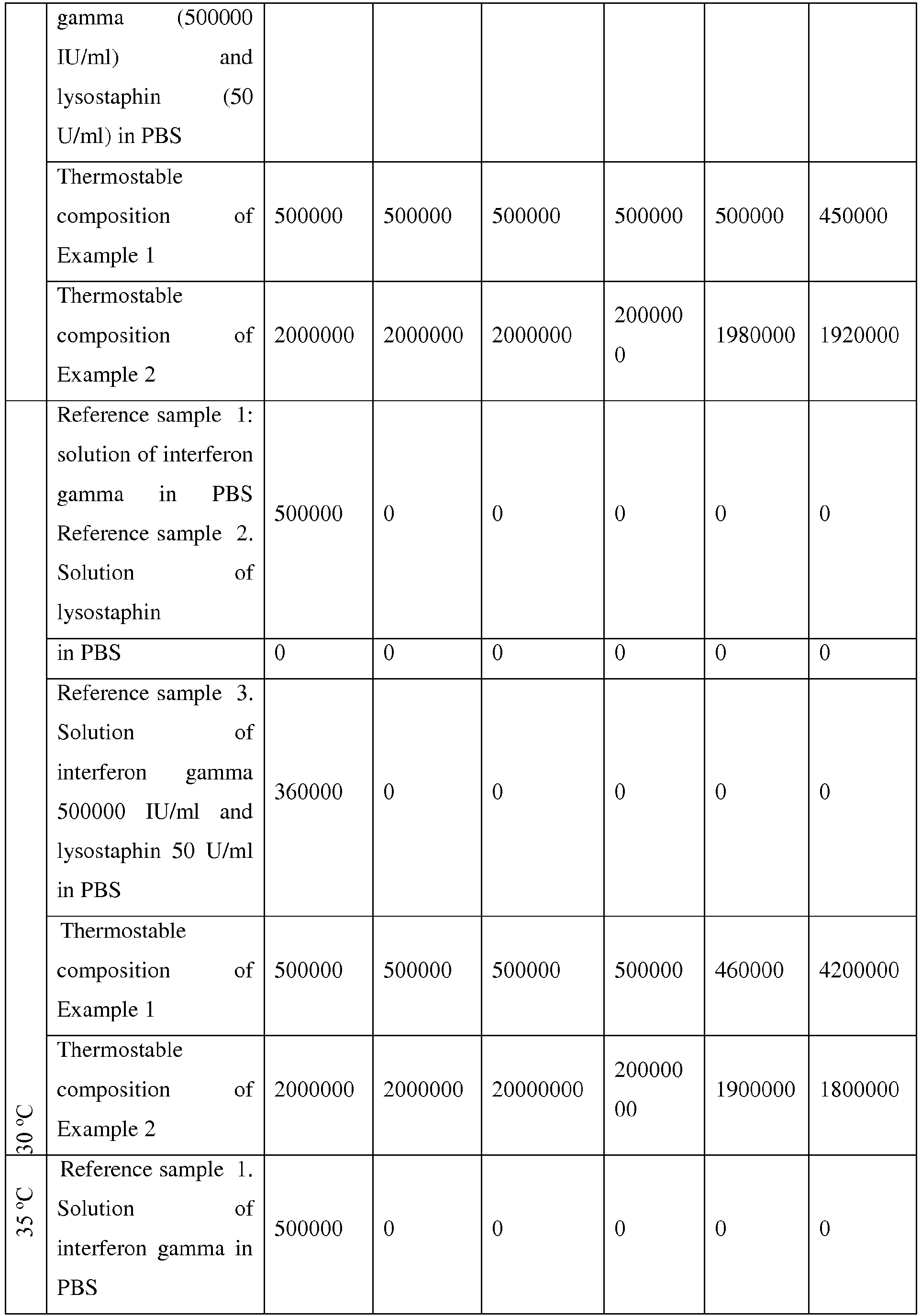

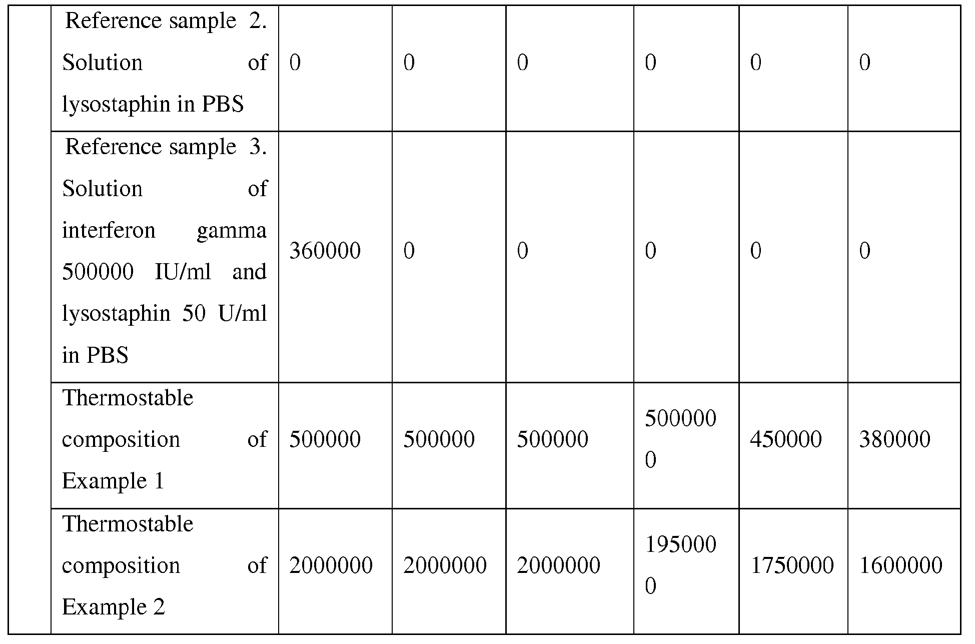

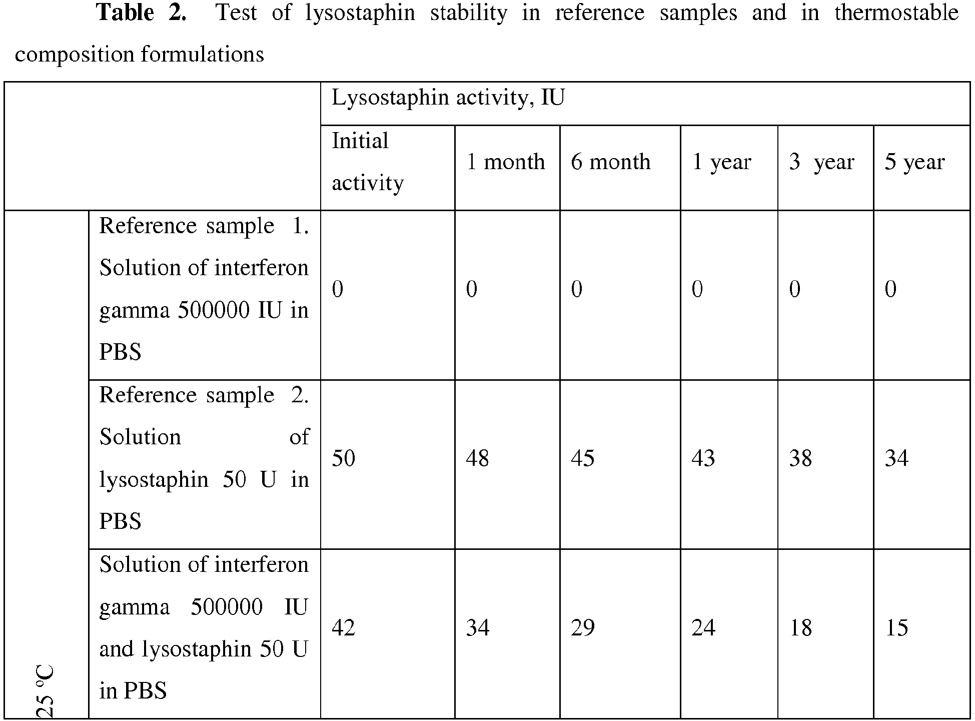

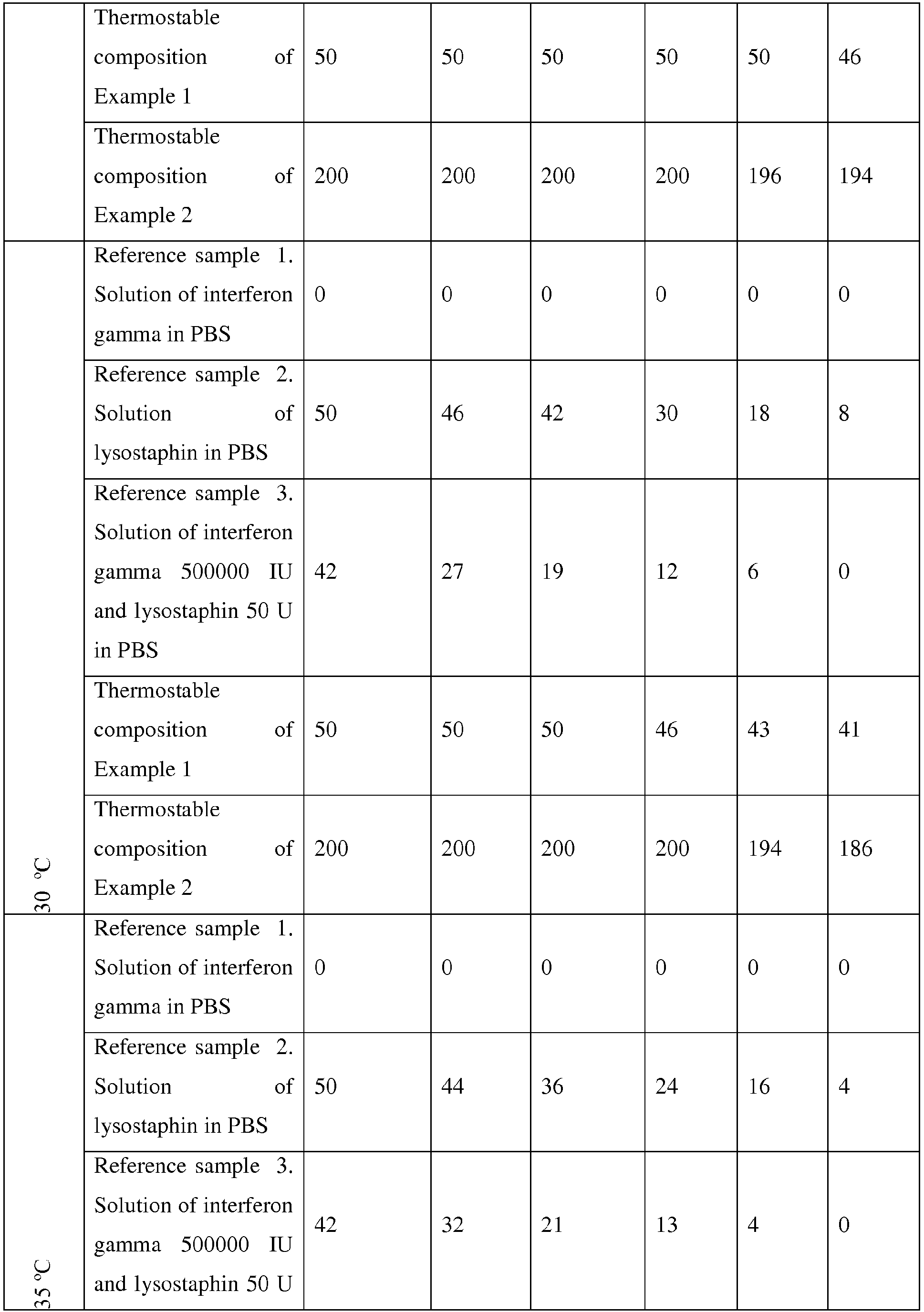

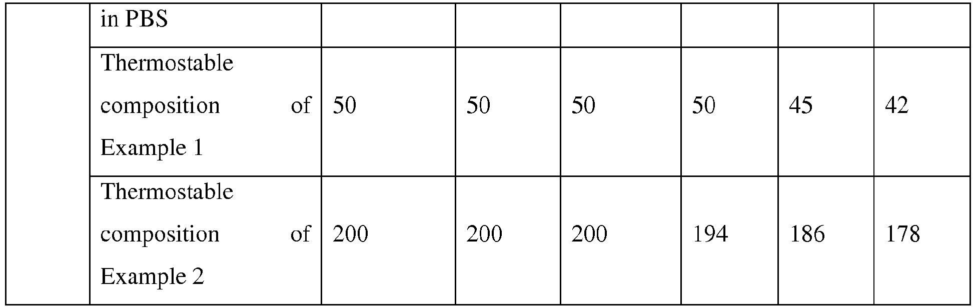

Results of these tests have demonstrated that:
1. The initial activity of the prepared test solutions corresponds to the expected and is equal in value to that of the reference samples containing any of the active substances under standard conditions.
2. A sample representing a mixture of recombinant interferon gamma human and lysostaphin without auxiliary ingredients showed activity values below the expected ones. It can be assumed that this activity loss is associated with interaction of the components. Moreover, control samples containing a simple mixture of recombinant interferon gamma human and lysostaphin completely lost their activity under all storage conditions in less than a month.
3. When stored under specified conditions during the first month, control samples containing only recombinant interferon gamma human lost up to 70% of the activity when stored in room conditions and completely lost the activity at temperatures of 30 ° C and 35 ° C regardless of the buffer composition.
4. When stored under specified conditions during the first month, control samples containing lysostaphin alone lost up to 30% of their activity when stored at room temperature, up to 90% when stored at 30 ° C and completely lost the activity after prolonged exposure to high temperature.
5. The thermostable compositions of Examples 1 and 2 retained the initial activity of their protein components during the first month under all temperature storage conditions.
t6. The Thermostable compositions retained their thermostability when stored at + 25 ° C and at + 30 ° C during the storage for 5 years. At the end of this period, when stored at 35 ° C, both compositions lost about 15% of their activity.

Thus, it has been unexpectedly found that the thermostable composition retains its properties when stored at ambient temperature up to + 25 ° C for 5 years, and at + 30 ° C it can be stored for at least 3 years.

Examples given below illustrate technological implementation of the proposed invention and can be reproduced by any expert skilled in this technology field. The given examples are merely illustrative, disclose various aspects of the possible implementation of the proposed invention and should not be interpreted as limiting the technology of the invention.

### EXAMPLES OF IMPLEMETING THE INVETION

### EXAMPLE 1. Preparation of the thermostable composition possessing antiviral and antibacterial activity.

Thaw recombinant interferon gamma human and lysostaphin substances to obtain the thermostable composition. Pour 500 ml of water into a container supplied with a stirring device. Mix the solution with methoxypolyethylene glycol, sodium alginate, sodium dihydrophosphate dihydrate, citric acid and dextran in the amount of 3 g, 1 g, 12 g 11 g and 73.0 g, respectively. Stir up the mixture and measure pH, which should be 5.0. Add 100 ml of the thawed-out lysostaphin substance with the activity of 50 U / ml to the dissolved ingredients and mix them up. Then add 17 ml of the thawed interferon gamma substance with an activity of 3.000.000 IU / ml.

Adjust the obtained volume to 1 L with water and stir for 15 minutes. Avoid foaming. Filter the resulting solution through 0.45-µm and 0.2-µm filters and pour it aseptically into 200-µl and 10-ml glass vials. Freeze the mixture at minus 40 ° C and lyophilize it for 36 hours. Seal the vials with rubber stoppers and aluminum caps.

The thermostable composition should represent a lyophilized white powder. The powder is hygroscopic. pH of the solution of one gram of the stabilized composition in 5 ml of water is 5.0

The values of antiviral and antibacterial activity are given in Tables 1 and 2. Use an in vitro test system based on Vero cell culture to test the antiviral activity. Use virus of vesicular stomatitis (VVS) as a test object. The dose of VVS should be 10 TCD₅₀. Dissolve 200 mg of the thermostable composition in 1 ml of water for injection to obtain a preparation sample. Use a reference strain of staphylococcus to test antibacterial activity. Adjust the cell suspension of the reference staphilococcus strain to a concentration of 1x105 cells / ml. Then prepare ten-fold dilutions at a concentration of 1x10² cells / ml. Dissolve 1 g of the thermostable composition in 10 ml of water for injection. Then add 1 ml of the resulting solution to 1 ml of the previously obtained suspension at various dilutions. Incubate for 10 minutes. Record the results spectrophotometrically at a wavelength of 600 nm.

The obtained lyophilized mixture should have interferon gamma and lysostaphin of the activity of 500000 IU / g and 50 U / g, respectively, and should contain the following components per 1 g of the end thermostable composition:
human recombinant interferon gamma
with activity of 500000 IU/g 0.03 mg
lysostaphin with activity of 50U/g0.17mg
methoxypolyethylene glycol 30 mg
sodium alginate 10 mg
   sodium dihydrophosphate dihydrate 120 mg
citric acid110 mg
dextran 730 mg.

Store the lyophilize composition at a temperature of + 25 ° C for 5 years. Control the activity during 5 years. After 5 years, the activity of interferon gamma and lysostaphin should be 90% 92% of the initial, respectively.

### EXAMPLE 2. Preparation of the thermostable composition possessing antiviral and antibacterial activity.

Thaw recombinant interferon gamma human and lysostaphin substances to obtain the thermostable composition. Pour 300ml of water into a container supplied with a stirring device. Mix the solution with methoxypolyethylene glycol, sodium alginate, sodium dihydrophosphate dihydrate , citric acid and dextran in an amount 15 g, 2.5 g, 25 g, 3.5 g, 54 g, respectively. pH of the solution should be 7.0. Add 400 ml of the thawed-out lysostaphin substance with the activity of 50 U / ml to the dissolved ingredients and mix them up. Then add 68 ml of the thawed interferon gamma substance with the activity of 3.000.000 IU / ml.

The substance "Recombinant interferon gamma human " is an aqueous solution of a protein synthesized by Escherichia coli SG 200-50 cells transformed with plasmid pGIF315.

Adjust the cell suspension volume to 1 l and stir it for 15 min. Avoid foaming. Filter the obtained solution through filters with pores of a size of 0.45 µm and 0.2 µm. Pour aseptically the solution into 200-mkl, 1-ml and 10-ml vials. Freeze it at minus 40°C and lyophilize, then dry the solution for 36 h and seal hermetically with rubber stoppers and aluminium caps.

The obtained lyophilized mixture should have interferon gamma and lysostaphin of an activity of 500000 IU / g and 50 U / g, respectively and should contain the following components per 1 g of the end product:
human recombinant interferon gamma
with activity of 2000000 IU/g 0.12 mg
lysostaphin with activity of 200 U/g 0.68 mg
methoxypolyethylene glycol 150 mg
sodium alginate 25 mg
   sodium dihydrophosphate dihydrate 250 mg
citric acid 35 mg
dextran 540 mg.

Store the lyophilized composition at a temperature of + 25 ° C for 5 years. Control the activity during 5 years. After 5 years of storage, the activity of interferon gamma and lysostaphin shoul be 90% and 92% of the initial activity, respectively.

### EXAMPLE 3. Preparation of the thermostable composition possessing antiviral and antibacterial activity.

Thaw recombinant interferon gamma human and lysostaphin substances to obtain the thermostable composition. Pour 300 ml of water into a container with a stirring device. Add methoxypolyethylene glycol, sodium alginate, sodium phosphate disubstituted dihydrate , citric acid and dextran in an amount of 9 g, 1.45 g, 16 g 8 g and of 65.5 g respectively. pH of the solution should be 6.0. Add 200 ml of the thawed substance of lysostaphin with the activity of 50 U / ml to the dissolved ingredients and mix up the solution. Then add the thawed substance of interferon gamma with the activity of 3.000.000 IU / ml in the amount of 34 ml.

The substance "recombinant interferon gamma human" is an aqueous solution of a protein synthesized by Escherichia coli SG 200-50 cells transformed with plasmid pGIF315.

Adjust the solution volume to 1 L with water and stir for 15 minutes. Avoid foaming. Filter the obtained solution through filters with pores of a size of 0.45 µm and 0.2 µm, and aseptically pour into 1-ml, 10-ml glass vials. Freeze the solutions at minus 40 ° C and lyophilize. Drying time should be 36 hours. Seal the vials with rubber stoppers and aluminum caps.

Pour aseptically the solution into 200-mkl, 1-ml and 10-ml vials. Freeze it at minus 40°C and lyophilize, then dry the solution for 36 h, seal the vials hermetically with rubber stoppers and aluminium caps.

The thermostable composition is a hygroscopic lyophilized white powder. pH of 1 g of the stabilized composition dissolved in 5 ml of water should be 6.0.

The obtained lyophilized mixture should have interferon gamma and lysostaphin of the activity of 100000 IU / g and 100 U / g, respectively, and should contain the following components per 1 g of the end product:
recombinant interferon gamma human
with activity of 1000000 IU/g 0.06 mg
lysostaphin with activity of 100 U/g 0.425 mg
methoxypolyethylene glycol90 mg
sodium alginate 15 mg
   sodium dihydrophosphate dihydrate 160 mg
citric acid 80 mg
dextran 655 mg

Store the lyophilized composition at a temperature of + 25 ° C for 5 years. Control the activity during 5 years. After 5 years of storage, the activity of interferon gamma and lysostaphin shoul be 90% and 92% of the initial activity, respectively.

### EXAMPLE 4. An oral cavity care product formulated as an aqueous solution.

Prepare the product from the thermostable composition as described in Example No. 2, wherein interferon gamma and lysostaphin activity is 2.000.000 IU / g and 200 U/g, respectively. To prepare the product dissolve 20 mg of lyophilizated thermostable composition in 200 ml of water, stir the solution until completely dissolved. The product represents a colorless liquid with pH 7.0 and activity of interferon gamma and lysostaphin being 200 IU / ml and 0.02 U / m, respectively.

The resulting solution is used for rinsing oral and nasal cavities. The product is not toxic and does not cause side effects.

### EXAMPLE 5. An oral cavity care product formulated as a rinsing liquid.

Weigh 300 mg of sodium chloride, 15 g of sorbitol, 3 g of sodium benzoate and dissolve each sample with 0.5 1 of water in separate vials and stir up the solution. Pour 5 1 of water in a stirrer and add the previously dissolved ingredients. Stir up the solution for 10 minutes. Diluted 1 g of the thermostable composition prepared as described in Example 2 and pour the solution into the stirrer. Avoid foaming. After 5 minutes, adjust the solution volume to 10.0 1 with water. pH should be 7.0. Stir up the solution for 5 minutes.

After sterilizing filtration, pour the solution aseptically into 150-ml flasks.

The product represents a colorless liquid, light opalescence is possible.

Activity of interferon gamma and lysostaphin in the product should be 200 IU / ml and 0.02 U / m, respectively.

The product is used for preventive purposes as well as to reduce the risk of various inflammations and infectious complications after medical and other intensive interventions in the oral cavity. The product is not toxic, does not cause side effects.

Application instruction: rinse the mouth with 50-60 ml of the product. Do not rinse with water afterwards and do not drink for 30 minutes. Use 3 - 5 times as needed, but at least 3 times a day. Shelf life: not less than 3 years when stored at room temperature.

The mouth rinsing product with interferon gamma and lysostaphin activity of 200 IU / ml and 0.02 U / ml, respectively, has the following components per 1 ml:
thermostable composition 0.1mg
sodium chloride 0.03 mg
sorbitol 1.5 mg
sodium benzoate 0.3 mg
waterup to 1 ml.

### EXAMPLE 6. An oral cavity care product formulated as a rinsing liquid.

Weigh 1.5 mg of sodium chloride, 7.5 g of sorbitol, 5 g of sodium benzoate and dissolve each sample in separate vials in 0.5 1 of water and stir up the solution. Pour 5 1 of water into the stirrer and add the previously dissolved ingredients. Stir up the solution for 10 minutes. Diluted 20 g of the thermostable composition prepared as described in Example 1 and pour the solution into the stirrer. Avoid foaming. After 5 minutes, adjust the solution volume to 10.0 1 with water. pH should be 5.0. Stir up the solution for 5 minutes.

After sterilizing filtration, pour the solution aseptically into 150-ml flasks.

The product represents a colorless liquid, light opalescence is possible.

Activity of interferon gamma and lysostaphin in the product should be 200 IU / ml and 0.02 U / m, respectively.

The product is used for preventive purposes as well as to reduce the risk of various inflammations and infectious complications after medical and other intensive interventions in the mouth cavity. The product is not toxic, does not cause side effects.

Application instruction: rinse the mouth with 50-60 ml of the product. Do not rinse with water afterwards and do not drink for 30 minutes. Use 3 - 5 times as needed, but at least 3 times a day. Shelf life: not less than 3 years when stored at room temperature.

The oral cavity rinsing liquid with interferon gamma and lysostaphin activity of 200 IU / ml and 0.02 U / ml, respectively has the following components per 1 ml:
thermostable composition 0.1mg
sodium chloride 0.03 mg
sorbitol 1.5 mg
sodium benzoate 0.3 mg
water up to 1 ml.

### EXAMPLE 7. An oral cavity care product formulated as a spray.

Weigh 600 mg of povidone, 30 mg of potassium sorbate, 3 g of sodium benzoate and dissolve each sample in separate vials in 0.5 1 of purified water and stir up the solution. Pour 5 1 of water into the stirrer and add the previously dissolved ingredients. Then add 60% solution of potassium lactate in a volume of 0.3 ml. Stir up the solution for 10 minutes. Dilute 2 g of the thermostable composition prepared as described in Example 3 and pour the solution into the stirrer. Avoid foaming. pH should be 6.0. Add 10 mg of peppermint oil. Then adjust the solution volume to 10.0 1 with water. After 10 minutes, turn off the stirrer.

After sterilizing filtration, pour the solution into flasks supplied with a sprayer.

The product represents a liquid with low viscosity and light opalescence. The product has a specific pleasant flavor and taste.

Activity of interferon gamma and lysostaphin in the product should be 200 IU / ml and 0.02 U / m, respectively.

The product is intended for hygienic, prophylactic and sanitary mouth care and also for reducing the risk of inflammations and infectious complications after medical and other intensive interventions into an oral cavity. The product is not toxic and does not cause side effects.

Application instruction: rinse the oral cavity directing the spray to the problem areas. Do not rinse with water afterwards and do not drink for 30 minutes. Use 3 - 5 times as needed, but at least 3 times a day. Shelf life: not less than 3 years when stored at room temperature.

The oral cavity rinsing product with interferon gamma and lysostaphin activity of 200 IU / ml and 0.02 U / ml, respectively, has the following components per 1 ml:
thermostable composition0.2mg
povidone K300.06mg
potassium sorbate0.003mg
potassium lactate0.02mg
peppermint oil 0.001mg
sodium benzoate 0.3mg
waterup to 1 ml.

### EXAMPLE 8. An oral cavity care product formulated as a spray.

Weigh 600 mg of povidone, 30 mg of potassium sorbate, 3 g of sodium benzoate and dissolve each sample in separate vials in 0.5 1 of purified water and stir up the solution. Pour 5 1 of water into the stirrer and add the previously dissolved ingredients. Then add 60% solution of potassium lactate in a volume of 0.3 ml. Stir up the solution for 10 minutes. Dilute 3 g of the thermostable composition prepared as described in Example 2 and pour the solution into the stirrer. Avoid foaming. pH should be 7.0. Add 10 mg of peppermint oil. Then adjust the solution volume to 10.0 1 with water. In 10 minutes, turn off the stirrer.

After sterilizing filtration, pour the solution into flasks supplied with a sprayer.

The product represents a low-viscosity liquid with light opalescence, a specific pleasant flavor and taste.

Activity of interferon gamma and lysostaphin in the product should be 600 IU / ml and 0.02 U / m, respectively.

The product is intended for hygienic, prophylactic and sanitary mouth care and also for reducing the risk of inflammations and infectious complications after medical and other intensive interventions in the oral cavity.

Application instruction: rinse the oral cavity directing the spray to the problem areas. Do not rinse with water afterwards and do not drink for 30 minutes. Use 3 - 5 times as necessary at least 3 times a day. Shelf life: not less than 3 years when stored at room temperature.

The spray for oral cavity care with interferon gamma and lysostaphin activity of 600 IU / ml and 0.02 U / ml, respectively, has the following composition per 1 ml:
thermostable composition 0.3 mg
povidone K30 0.18 mg
potassium sorbate 0.018 mg
potassium lactate 0.06 mg
peppermint oil 0.003 mg
sodium benzoate 0.9 mg
water up to 1 ml.

### EXAMPLE 9. A toothpaste.

Weigh 50 mg of sodium fluoride, 0.5 g of detergent, 1 g of xylitol, 0.5 g of sodium hydrogen carbonate, 50 mg of sodium benzoate to prepare 100 g of the toothpaste. Use silicon oxide as mild abrasive filler in an amount required for preparation of 100 g of the paste. Mix the weighted ingredients, add 50 mg of the thermostable composition prepared as described in Example 1 to 1/10 of the obtained mixture. Stir the mixture up and add it to the remaining 9/10 parts.

Place the paste in 40-ml aluminum tubes with screw caps.

Prepare samples as follows to control the product activity. Weigh 1 g of paste and shake it in 5 ml of purified water. Filter the mixture through a paper filter. After sterilizing filtration through a filter with the pores of a size of 0.22 µm, perform testing as described in Example 1.

The antiviral activity of interferon gamma and lysostaphin in 1 g of toothpaste should be 200 IU / g and 0.02 U / g, respectively.

The toothpaste is used as a preventive product for gums and tooth brushing and for lowering the risk of inflammations and infectious complications after medical and other intensive interventions in the oral cavity cavity. Use the paste as needed.

Shelf life of the paste is 3 years.

### EXAMPLE 10. A toothpaste.

Use calcium carbonate with 10% dicalcium phosphate dihydrate as a basic substrate. Add also strontium chloride, glycerol, sodium benzoate, a detergent, xylitol, sodium bicarbonate and sodium benzoate in an amount of 20 mg, 0.5 g, 50 mg, 0.5 g , 1 g, 0.5 g and 50 mg, respectively, as suitable substrates. Add 50 mg of the thermostable composition prepared as described in Example 2 to 1/10 of the obtained substrate and mix them thoroughly. Add the remained substrate to the resulting mixture gradually.

The antiviral activity of interferon gamma and lysostaphin in 1 g of the toothpaste should be 1000 IU / g and 0.1 U / g, respectively.

The toothpaste is used as a prophylactic product for gums and tooth brushing and for lowering the risk of inflammations and infectious complications after medical and other intensive interventions in the oral cavity. Use the paste as needed.

Shelf life of the paste is 3 years.

### EXAMPLE 11. A sanitary-hygienic product.

Use cotton fabric as a base. Apply 15 mg of the thermostable composition on each square cm of the fabric sample. Use the thermostable composition prepared as described in Example 1. Place the obtained sample in a horizontal position, dry it under normal conditions and then roll the sample with a hard rubber roller. Continue drying for 24 hours at room temperature.

In the obtained sanitary-hygienic product the activity of interferon gamma and lysostaphin is 5000 IU / cm², 0.5 U / cm², respectively.

Cut the product samples into pieces of a size of about 4 cm² and 10 cm². Place the samples of a size of 4 cm² under sterile conditions in the suspension of *Staphylococcus* culture with 1x10⁷ microorganism cells per 1 ml with an optical density 1 OD unit at a wavelength 600 nm.

Wash the product sample without stirring in a test tube with *Staphylococcus* suspension for 10 minutes (use a sterile forceps). Continue testing as described in Example 1.

Test results should be in compliance with the abovementioned levels of activity.

Evaluate the level of antiviral activity after elution of the active components from the substrate. Then filter the mixture through a 0.22-µm filter as described in Example 1.

Sanitary and hygienic product is designed to remove biological or external liquid from the surface of skin. The product has sufficient ability to absorb moisture while not only mechanically cleaning the skin surfaces from contamination, but also effectively eliminating microbial contamination. Holding moisture collected from skin surface and contaminations inside the thickness of woven fabric, the product also disinfects the polluted fluid that facilitates utilization of the product as an ordinary household waste. The product used is not a source of secondary infection.

Shelf life of the product is 3 years.

### EXAMPLE 12. A sanitary-hygienic product.

Use the thermostable composition prepared as described in Example 2 to prepare a sanitary-hygienic product sample. Use a fabric containing 60% cotton and 40% synthetic fibers as a substrate.

Moisten the woven fabric with water. Weigh the thermostable composition at a rate of 15 mg per cm². Dissolve the sample in purified water (1 ml per 100 mg of the thermostable composition) under low stirring rate. Allow the solution of the thermostable composition to stand for swelling for 30 minutes and then apply it eventually over the surface of the woven fabric and dry at room temperature for 24 hours.

The activity of interferon gamma and lysostaphin in the sanitary-hygienic product should be 20,000 IU / cm² and 2 U / cm², respectively.

Select the test samples and control the activity of the main components as described in Example 11.

When the described technology of application is used, the degree of retention of the basic substances on the surface and in the thickness of the fabric is much higher than in Example 11. The amount of material that is not fixed on the fabric substrate after drying is less than 5%.

Analysis of the activity of the main components has shown that the activity of the surface washable and released components is about 85% of the initial. The test results have demonstrated the compliance with the abovementioned levels of activity.

The obtained sanitary-hygienic product is used as a wipe.

To produce a medical product in the form of gloves, a pattern of gloves was made from the obtained fabric. The product may well be used for short-term (no more than 2 hours) protection of hand skin from microbial and / or viral infection in situations in which the infection is possible, for example, during A.R.V.I, or influenza epidemics. The protective mechanisms are activated after moistening the gloves with body fluids or with moisture from the environment. Antimicrobial effect is achieved immediately after microorganisms appear on the surface of the product. Antiviral effect is achieved when viruses reach the skin surface along with the components of the formulation. The product also forms a mechanical barrier that prevents direct exposure of skin surface to microorganisms.

### EXAMPLE 13. A sanitary-hygienic product.

The following technique for application of the thermostable composition is proposed: treat the woven fabric with a filling agent. Then remove the excess of the filling agent and apply the solution of the thermostable composition onto the product surface.

First, prepare a solution containing 5 g of carboxymethylcellulose, 0.05 g of trehalose, 1 g of sodium alginate and 0.1 g of sodium benzoate. Use water as a solvent in the amount of 100 ml. Prepare the solution at room temperature with periodic stirring for 30 minutes until substrate is completely swollen.

Apply the resulting solution to the fabric base. Allow the mixture to stand for 20 minutes and remove the excess liquid.

Apply the solution of the thermostable composition described in Example 12 to the product sample. In comparison with Example 12, the solution of the thermostable composition is applied successively on both sides of the product with preliminary drying between the applications.

After applying the solution of the thermostable composition, dry the product for 20 hours in an air flow to residual moisture of not more than 7% by weight.

The activity of interferon gamma and lysostaphin in the sanitary-hygienic product is 20.000 IU / g and 2 units / g, respectively.

Use of the proposed application technique allows an efficient utilization of the thermostable composition in the products having fabric or unwoven material base. At the same time, the method is labor-consuming and increases the prime cost of the product.

The proposed product is designed to remove contaminants and excessive moisture from the skin. Due to the fact that the active components of the thermostable composition are distributed on the surface of the product practically without penetrating into the substrate, the product is more effective in common use (relatively short wiping) than those described in Examples 11 and 12 as the higher amounts of the components are transferred on the treated surface. Consequently, a thin protective film formed on the surface allows for retaining the protective effect for some time after the use of the product. In this case, the product retains the same properties as those described in Examples 11 and 12, including the expiry date.

### EXAMPLE 14. A sanitary-hygienic product.

Use the product described in Example 12 to make a conventional medical mask.

Protein active ingredients do no exhibit their activity in dry state. However, the product in the form of a protective mask is directly in contact with the skin of a person and, therefore, at ambient temperature, is moistened with biological fluids, e.g. sweat. It is also moisturized by the water vapor contained in expiratory air that condenses on the fabric base of the medical mask.

In order to assess the efficacy of the product "medical mask" use a finely dispersed agent to simulate an airborne droplet contamination of the outer surface of the product prepared as described in Example 12. Spray 0.5 ml of microorganism suspension at a concentration of 10³ microbes per 1 ml into 12 products independently. Moisten the product from the inside with a sprayer. Perform testing as described in Example 12.

Testing results have shown that the forced infection with a test strain at high doses is completely blocked by the product due to its biocidal effect. The surface of the Petri dishes remains clean with no growth of colonies while the test samples (positive control) exhibit the expected growth. Negative control (the level of background dissemination) has not demonstrated any growth, that supports validity of the test. The test results have shown a compliance with the above levels of activity.

An ordinary medical mask, including a multi-layered one, creates only a mechanical barrier that traps the dust particles and water microdrops, through which dangerous viruses and microorganisms are spread. In this regard, the operating time of the conventional medical mask is most often limited to 2 hours. In conditions of severe pollution, this time decreases.

When using the medical mask prepared in this Example, the antiviral and antimicrobial effect is supplemented with the property of mechanical retention of external particles, as a result of which the operating time increases significantly and is determined mainly by the need of usage and dust content in the external environment. At the same time, the longer the mask is used the more components are transferred to the skin surface. These, in turn, form a thin polymer protective film in the sites of contact with skin surfaces that enhances the overall positive effect of the product.

Place the prepared product in an individual hermetically sealed pack and store at room temperature for 3 years. Control the product at the end of the storage period.

Activity testing of the main components has shown that the antiviral and antimicrobial activity of interferon gamma and lysostaphin is reduced by 7% and 4%, respectively, in comparison with the initial one.

### EXAMPLE 15. A sanitary-hygienic product on a paper base.

This example differs from Example 11 by use of a one-layer low density cellulose paper as the base.

Perform the activity test as described in Example 11. Dry the product the airflow up to residual moisture of 7% wt.

The activity of interferon gamma and lysostaphin should be 5000 IU / cm² and 0.5 U / cm², respectively

Test the antiviral activity of the product as follows: cut a test sample of a size of 100 cm², fold it into a loose roll and place in a container with 10 ml of purified water. Stir the sample for 15 minutes and then remove it from the solution. Use a forceps. Filter the resulting solution through a "blue ribbon" paper to remove large residues of paper detached from the sample base. Sterilize the solution by passing it through a 0.22-µm filter. Perform further testing as described in Example 1.

Make dry disposable handkerchiefs, disposable dry wipes and paper towels of the product obtained. The test results have shown a compliance with the abovementioned levels of activity.

The product is used for wiping skin and can be utilized through a household sewage.

Shelf life of the product is 3 years.

### EXAMPLE 16. A sanitary-hygienic product.

Prepare the product as described in Example 15. Package the obtained paper napkins both individually or in packs, 100 items each.

The activity of interferon gamma and lysostaphin in the product is 20000 IU / g and 2 U / g, respectively.

### EXAMPLE 17. A medical product (sponge).

When producing a sponge, apply the thermostable composition on extruded cellulose substrate. Use porous, semi-rigid cutting sheets with dimensions (500x1000) mm as the substrate and cut them into pieces after application of the thermostable composition.

The prepared product represents a sterile hard fibrous hydrophilic sponge of a size of 5x5 cm and thickness from 2 to 7 mm for topical application. It may be packaged individually or in packs, 5, 10, 20 or more items each. The activity of interferon gamma and lysostaphin in the product is 5000 IU / g and 0.5 U / g, respectively.

The product possesses biocidal activity and may be used for treatment of skin, in particular skin with staphylococcal or herpes lesions. In light cases of skin lesions, the product can be used alone or in combination with medicines for external application. The product may be fixed with a bandage or adhesive plaster.

The product has good moisture absorbing capacity. After moistening with water or biological fluids at the site of contact with skin lesions, it forms a stable protective film, from which the active components are gradually released. This increases topical effect of the product. The test results have shown a compliance with the abovementioned levels of activity.

If necessary, the residues of the protective film can be easily removed from the skin. The product does not cause a local reaction. It is not allergenic. The shelf life of the product is 3 years when stored in a dry place.

### EXAMPLE 18. A sanitary product (wipe).

Cut the sanitary-hygienic product obtained as described in Example 13 into pieces of a size of 40 x 40 cm.

Modify the efficiency analysis procedure as follows:
Make a mark of the sterile surface of a size of 10 x 10 cm². Use 4 dilutions of the test microorganism in 12 squares. Then mark 6 more squares of the same area. Apply the dilutions of the test microorganism with a cotton tampon attached to a wooden stick. Preliminary wet the tampon in cell suspension. Leave the dilutions for drying (no visible traces of moisture should be noticed).

Fold the wipe twice and wipe ¼ of the squares with different dilutions. Do not press the wipe strongly. Each square should be treated only once with one pleat of the folded wipe.

Expose the squares for 10 min after wiping and place then the remaining microorganisms to Petri dishes by a fingerprinting method.

Infect the remaining squares with Staphylococcus reference strain. Do not wipe the squares. Use a fingerprinting method to transfer the microorganisms from the test surface to Petri dishes with a culture medium. Place the dilution of microorganisms with a cotton tampon attached to a wooden stick directly onto Petri dishes to determine the level of the initial infection of squares on the test surface. Preliminary moisten the tampon with the culture liquid of the test strain.

It has been found that antimicrobial effect of the wipes when used on the test surfaces provides almost 100% disinfection of the surfaces in all tests. This is due to the fact that poorly attached microorganisms are mechanically removed from the surfaces and fall into the depth of the wipe fabric where they die; better attached microorganism lose their viability due to lysostaphin action. The used wipes do not become a source of secondary contamination and do not require special utilization measures. Test results correspond to the abovementioned activity levels.

Shelf life of the product is 3 years.

### EXAMPLE 19. A sanitary-hygienic wet product.

Use a cotton fabric as a base. Apply the thermostable composition prepared as described in Example 1 to the fabric sample (200 mg of the composition per 1 square cm of the fabric). Placed the fabric sample horizontally, dry it preliminary in ambient conditions for 24 h and roll with a hard rubber roller.

The prepared wet product contains interferon gamma and lysostaphin of the activity of 4000 IU / cm² and 0.4 U / cm², respectively.

Stop drying when the residual moisture content is 15% by weight. Test results have shown a compliance with the abovementioned activity levels.

Shelf life of the product is 3 years.

### EXAMPLE 20. A sanitary-hygienic wet product.

Use the thermostable composition prepared as described in Example 1. Use a mixed fabric containing 60% cotton and 40% synthetic fibers as the substrate.

Use the following additional components to prepare a filling agent: 20 g of carboxymethylcellulose, 10 g of sodium alginate and 0.5 g of sodium benzoate. Use water as a solvent in the amount of 100 ml. Prepare the solution at room temperature with periodic stirring for 30 minutes until the filling agent is completely swollen.

The obtained filler has a gel-like structure. Prepare the filler in a water bath at 50 ° C to preserve its mobility. Apply the filler base to a fabric consisting of cellulose and viscose fibers as described in Example 13. In contrast to the procedure described in Example 13, apply the filler with a rubber roller and at a temperature of about 40 ° C. Wring out the obtained filler and dry to a moisture content of 20% by weight.

Activity of gamma interferon and lysostaphin is 16000 IU / cm² and 1.6 U / cm², respectively.

The product is intended for topical use either alone or with fixing materials, e.g. medical bandage or another suitable product for skin attachment. The product forms a stable biologically safe film on the skin surface. This leads to even more pronounced disinfecting effect compared to the product for wiping. Test results correspond to the abovementioned activity levels. Shelf life of the product is 3 years if stored in a hermetic package.

### EXAMPLE 21. A disposable absorbent product.

Cut the sanitary-hygienic product prepared as described in of Example 11 into pieces of a size of 20x20 cm. Add cotton to the product and fold it with the edges being inside the product. Do not unfold the product when using.

At the same time, the ratio cotton: product should be 5: 1by weight.

Use the product for topical application.

Shelf life of the product is 3 years.

Use the product to remove large quantities of liquids of natural or biological origin, in particular for removal of exudates.

### EXAMPLE 22. A disposable absorbent product (tampon).

Use medical hygroscopic cotton as moisture absorbing filler. Treat the cotton preliminary to increase fiber density. Weigh 5 g of cotton and make a tampon by a mechanical way. Place cotton inside the sanitary hygienic product as described in Example 11. Tightly wrap the tampon with four layers of the sanitary hygienic product. Fold the edges of the fabric inside the tampon and fixed them with a thread to prevent spontaneous opening. The ratio of the cotton and the product is 10: 3 by weight.

### EXAMPLE 23. A disposable hygienic product (hygienic liner).

Prepare a disposable hygienic product as described in Example 22. Make a liner in the form of rectangle of a size of 4x6 cm and a thickness of 1 cm. Make a pattern of the product as described in Example 12 to obtain an easy-to-use hygienic liner with "wings ". Then place a cotton tampon inside the pattern, fold the edges and stitch them mechanically. As an option, use glue to attach the edges. The weight-to-weight ratio of cotton and the liner is 9: 2.

### EXAMPLE 24. A disposable hygienic product (daily liner).

Prepare a disposable hygienic product as described in Example 22. Treat preliminary a cotton filler with a flavoring agent. The thickness of the cotton absorbent filler should be 0.5 cm. Before applying the sample on the pattern described in Example 12, place a semisynthetic semipermeable unwoven material on both sides of the cotton absorbent liner to prevent the discharge of moisture. The weight-to-weight ratio of cotton and the product is 5: 2.

Place the product into polyethylene film packs, 10 items each.

The product can be adapted for different conditions of use by adjustment of the thickness of moisture-absorbing layer. Addition of silica gel and unwoven materials with adhesive layer for attachment of the product to clothes might be beneficial for consumer properties of the product.

### EXAMPLE 25. A skin care product.

In order to obtain the claimed skin care product prepare a cosmetically acceptable base containing (mass%):
vegetable oil 12
stearate 2 4.2
stearate 214.2
cetyl stearyl alcohol3.1
glycerol4
propylene glycol3.5
lanoline 6
mixture of vitamines A and E1.3
cacao butter 4.2
preservative 2
vanilla flavor 0.3
water up to 100

Mix the obtained cosmetically acceptable base in the amount of 100 g with 50 g of the thermostable composition with interferon gamma and lysostaphin activity of 100,000 IU / g and 100 U / g, respectively, as described in Example 3.

Pack the end product representing a cosmetic face mask into suitable containers (for example, tubes), seal them and attach labels.

The obtained product has an activity of interferon gamma 500 IU / g, and that of lysostaphin 0.05 U /g.

Shelf life of the product is 3 years.

### EXAMPLE 26. A skin care product.

Prepare the product using the thermostable composition containing interferon and lysostaphin of the activity of 500.000 IU / g and 50 U / g, respectively, as described in Example 1.
1 g of the product contains:
thermostable composition 4 mg
spermacet 100 mg
sodium benzoate 0.1 mg
sodium chloride 0.3mg
beta-carotene 0.2mg
vaseline 10 mg
lanoline 190 mg
cacao butter700 mg.

The obtained product has the activity of interferon gamma 2000 IU / g, and that of lysostaphin 50 U / g.

The product formulated as a cream can be used for hygienic, preventive and revitalizing skin care in case of inflammation, purulent skin or furuncles. The product does not cause side effect, it is not toxic, has biocidal effect and is beneficial for skin texture.

Shelf life of the product is 3 years.

### EXAMPLE 27. A skin care product.

Use the thermostable composition containing 500,000 IU / g of interferon gamma and 50 U / g of lysostaphin as described in Example 1 to prepare the product in the form of gel. Prepare a filler substrate containing 20 g of carboxymethylcellulose, 10 g of sodium alginate and 0.05 g of sodium benzoate as additional component. Use water in an amount of 100 ml as a solvent. Prepare the solution at room temperature with periodic stirring for 30 minutes until the filler substrate is completely swollen. Then add 1 mg of the thermostable composition to 1 g of the filler. The obtained product should represent a dense gel.

In the obtained product the activity of recombinant interferon gamma human and lysostaphin is 500 IU / g and 0.05 U / g, respectively.

The cosmetic gel forms a thin biodegradable film that gradually releases active ingredients throughout the contact period on the surface of the treated skin. The components used have good biodegradability, exhibit hemostatic, absorbing and other therapeutic properties that makes the gel on the base of them attractable for preventive and hygienic skin care.

The product can be used for hygienic, preventive and health-improving skin care for humans and animals with inflammation, suppuration, furunculosis and other manifestations of bacterial infection.

The product is safe for humans and animals.

Application instruction: use a dispenser to squeeze out the product on a spatula (or cotton tampon) and evenly distribute it on problematic areas of the skin. Use as needed, but at least 2 times a day. Duration of the course is from 2 to 3 weeks.

Assess the efficacy of the product as described in Example 1. Test results should show the compliance of the activity levels.

Package the obtained gel into 10-, 25- and 50-g vials. Shelf life of the gel is 3 years.

### EXAMPLE 28. A skin care product.

Use gelatin, agar-agar, tragant and starch in a quantity of 3 g, 2 g, 1 g and 2 g, respectively, to prepare an acceptable cosmetic substrate in the form of jelly. Add the dry substances to the previously prepared aqueous glycerol mixture consisting of 30 ml of glycerin and 50 ml of purified water under constant stirring. Then add 0.1 g of the thermostable composition prepared as described in Example to 1/10 part of the prepared substrate and mix them. Then add the remaining aqueous glycerol mixture and mix again. As a result, 100 ml of the prepared jelly should be obtained.

Package the product in 25-g jars.

In the obtained product the activity of recombinant interferon gamma human and lysostaphin is 500 IU / g and 0.05 U / g, respectively.

The obtained product can be used for cosmetic purposes to treat skin with minor inflammatory foci, acne and other skin defects.

Shelf life of the product is 3 years.

### EXAMPLE 29. A skin care product.

Prepare water/glycerol mixture comprising 30 parts of glycerol and 50 parts of purified water to obtain a suitable cosmetic substrate in the form of gel. Weigh 2 g of PEG40, 3 g of gelatin and 5 g of carboxymethylcellulose. Add the dry substances to the previously prepared mixture under constant stirring. Then add 0.3 g of the thermostable composition prepared as described in Example 1. Mix thoroughly. Package the obtained cosmetic jelly in 25-g jars.

In the obtained product the activity of recombinant interferon gamma human and lysostaphin is 1500 IU / g and 0.15 U / g, respectively.

Shelf life of the product is 3 years.

### EXAMPLE 30. A skin care product.

Weigh 3 g of carbopol ultrez-21, 5 g of sorbite, 5 g of manuol, 5 g of cosmetic lavitol 5 g, 2 g of sea buckthorn seed oil to prepare a cosmetic product in the form of mask. Use 100 ml of water as a solvent. Add 0.1 g of the thermostable composition prepared as described in Example 2 to the obtained solution. Package the end product in individual packs, 20 items each.

In the obtained product the activity of recombinant interferon gamma human and lysostaphin is 2000 IU / g and 0.2 U / g, respectively.

Shelf life of the product is 3 years.

The product obtained is intended for application on face skin for 15 -60 minutes. It effectively softens a face skin, enhances antiviral immunity at a cell level, kills microorganisms and cleans cutaneous pores. It also has softening effect.

### EXAMPLE 31. A sanitary-hygienic product in the form of spray.

Weigh 200 g of trehalose, 4 g of potassium sorbate, 30 g of PEG400 and dissolve each sample in a separate container in 0.5 1 water. Use a magnetic stirrer for mixing. Pour 5 1 of water into the reactor and subsequently dissolve the ingredients. Stir the solution for at least 10 minutes. pH should be 6.0. Use triethanolamine to adjust pH of the solution. Dilute 10 g of the thermostable composition prepared as described in Example 2 in a small volume of water and add to the solution in the reactor. Avoid foaming. Then add 5 mg of lemon oil. Use water to bring the solution volume to 10.0 liters. After 10 minutes, turn off the reactor stirrer.

After sterilizing filtration, pour the product into vials supplied with a sprayer.

The product is applied as aerosol for spraying surfaces with high probability of dessimination with bacterial pathogenic microflora and skin surface, which requires special purity and additional protection from contamination. Shelf life of the product is not less than 3 years when stored at room temperature.

The resulting spray is used for intensive hygienic, preventive and health-improving skin care for animals with severe inflammation, suppuration, furunculosis and other manifestations of bacterial infection. It is used by aerosol spraying of the product on the skin of the animal. Use the product as needed at least 2 times a day. Duration of the course is from 2 to 3 weeks.

Also, the product can be used for intensive hygienic and preventive care for clothes to reduce the risk of infection and development of various inflammations and infectious diseases in humans wearing the infected clothes.

Application instruction: spray the product onto the clothes.

In the obtained product the activity of recombinant interferon gamma human and lysostaphin is 500 IU / g and 0.05 U / g, respectively.

1 ml of the product contains:
thermostable composition 1mg
trehalose 20 mg
triethanolamineto adjust pH to 6.0
potassium sorbate0.4mg
PEG400 3mg
lemon oil 0.5mg
waterup to 1 ml.

### EXAMPLE 32. A sanitary-hygienic product in the form of spray.

Prepare samples of 500 g trehalose, 150 g potassium sorbate, 150 g PEG 400 and dissolve each sample in a separate container in 0.5 1 water. Use a magnetic stirrer for mixing. Pour 5 1 of into the reactor, then subsequently introduce the dissolved ingredients. Stir the solution for at least 10 minutes. pH should be 7.0. Use triethanolamine to adjust pH. Add 500 g of the thermostable composition prepared as described in Example 2. Avoid foaming. Add 5 mg of peppermint oil. Then adjust the volume to 10.0 1 with water. After 10 minutes, turn off the reactor stirrer.

After sterilizing filtration, pour the product into vials supplied with a sprayer.

The product is applied as aerosol for spraying surfaces with high probability of dessimination with bacterial pathogenic microflora and skin surface, which requires special purity and additional protection from contamination. Shelf life of the product is not less than 3 years when stored at room temperature.

In the obtained product the activity of recombinant interferon gamma human and lysostaphin is 100000 IU / g and 10 U / g, respectively.

The product has the following composition:
thermostable composition 50 mg
trehalose 50 mg
triethanolamine to provide pH 7.0
   potassium sorbate 15mg
   PEG 400 15mg
peppermint oil 1.5mg
waterup to 1 ml.

### EXAMPLE 33. A sanitary-hygienic product for surface treatment.

Use the thermostable composition prepared as in Example 1 to prepare the product. The thermostable composition should contain interferon gamma and lysostaphin with the activity of 500.000 IU of and 50 U, respectively. Use 10 g of carboxymethylcellulose, 2 g of sodium alginate and 0.05 g of sodium benzoate to prepare a filling base. Use water in the amount of 100 ml as a solvent. Prepare the solution at room temperature with periodic stirring for 30 minutes until the filler is completely swollen.

Put the prepared gel into 10-, 25- and 50- gram jars.

Shelf life of the gel is 3 years.

Assess antimicrobial activity of the prepared gel as described in Example 13. The initial antimicrobial activity of the hygienic product should make 0.05 U / ml.

Use four samples of the hygienic product, 1 ml each, to control the antiviral activity as described in Example 1. The initial antiviral activity of the product should be 500 IU / ml.

The final antiviral activity of the product after 3 years of storage under standard conditions corresponds to that described in Examples 1-4.

The product is sufficiently fluid and after drying it forms a thin, relatively strong polymer film that provides prolonged release of the active ingredients. If needed, the film can be easily removed from the surfaces with water.

### EXAMPLE 34. A clothes conditioner.

Use the thermostable composition with the activity of 1,000,000 IU / g for interferon gamma and 100 U / g for lysostaphin described in Example 3 to prepare the conditioner.

Use the following components (per 1 ml of the product)

| | |
|---|---|
| thermostable composition | 10 mg |
| sodium benzoate | 0.4 mg |
| carboxymethyl cellulose | 5 mg |
| TWIN 20 | 5 mg |
| EDTA | 0.02 mg |
| water up to 1 ml. | |

Liquid formulation that provides better dispersion is preferable. The product can be delivered with a dosage device, e.g. sprayer.

Activity of interferon gamma and lysostaphin is 10000 IU/ml and 1 U/ml, respectively.

The clothes conditioner proposed in the invention should be used in a rinsing process without use of agents containing ionic detergents. Before application, the conditioner is diluted with water. The clothes thus treated are efficient when skin is infected with staphylococcus.

Shelf life of the product is 3 years.

### EXAMPLE 35. A clothes conditioner.

According to the invention, the conditioner should be used in a rinsing process without use of agents containing ionic detergents. Before application, the conditioner is diluted in aqueous solution.

Use the thermostable composition with the activity of interferon gamma and lysostaphin 2.000.000 IU / g and 200 U / g, respectively, as described in Example 2.

Use the following components (per 1 ml of the product):

| | |
|---|---|
| thermostable composition | 1 mg |
| sodium benzoate | 0.6 mg |
| carboxymethyl cellulose | 5 mg |
| TWIN 80 | 3 mg |
| EDTA | 0.02 mg |
| waterq.s. | |

In the product the activity of interferon gamma and lysostaphin is 2000 IU / g and 0.2 U / ml, respectively.

### EXAMPLE 36. A condom lubricant.

Use the thermostable composition with the activity of interferon gamma and lysostaphin 500.000 IU / g and 50 U / g, respectively, as described in Example 1 to prepare the product.

Dissolve the components to prepare the product. Add the components to a hydrophilic base comprising a mixture of polyethylene glycol 400 and polyethylene oxide 4000. Sterilize by autoclaving. Then add the thermostable composition and mix the solution. Package the product into suitable containers (for example, tubes), seal the containers and label them.

Take the components in the following ratio (per 1 g of the product):

| | |
|---|---|
| thermostable composition | 0.01mg |
| polyethylene glycol | 400 500 mg |
| polyethylene oxide | 4000 400 mg |
| glycerol | 100 mg |
| dimethyl sulfoxide | 0.002 mg |
| EDTA | 0.002 mg. |

In the obtained product the activity of recombinant interferon gamma human and lysostaphin is 5 IU / g and 0.0005 U / g, respectively.

The product is recommended for prevention of staphylococcal infections and sexually transmitted diseases.

### EXAMPLE 37. A condom lubricant.

Use the thermostable composition with the activity of interferon gamma and lysostaphin 1000000 IU / g and 100 U / g, respectively, as described in Example 1 to prepare the product.

Mix the components and sterilize by autoclaving. Then add the thermostable composition and mix. Package the product into suitable containers (for example, tubes), seal the containers and label them.

Take the components in the following ratio (per 1 g of the product):

| | |
|---|---|
| thermostable composition | 0.05 mg |
| farnesol | 0.00001 mg |
| glycerol | 100 mg |
| lanoline | 600 mg |
| dimethyl sulfoxide | 0.002 mg |
| cacao butter | 300 mg |
| vitamin E | 0.01mg |
| EDTA | 0.002 mg. |

In the obtained product the activity of recombinant interferon gamma human and lysostaphin is 5 IU / g and 0.0005 U / g, respectively.

Shelf life of the product is 3 years.

The product is recommended for prevention of sexually transmitted diseases.

### EXAMPLE 38. A medicinal product for treatment of skin infections in the form of ointment.

Use the thermostable composition with the activity of interferon gamma and lysostaphin 2000000 IU / g and 200 U / g, respectively, as described in Example 1 to prepare the product.

Take the components in the following ratio (per 1 g of the product):
thermostable composition 10 mg
spermacet 100 mg
sodium benzoate 0.1Mg
sodium chloride 5mg
Vaseline 150 mg
lanoline555 mg
cacao butter 150 mg.

In the obtained product the activity of recombinant interferon gamma human and lysostaphin is 20.000 IU / g and 2 U / g, respectively.

The product is used for skin treatment in case of inflammation, suppuration, furunculosis. The product does not cause side effects and is not toxic.

Shelf life of the product is 3 years.

### EXAMPLE 39. A medicinal product for treatment of skin infections in the form of gel.

The product in the form of gel is used to treat inflammation, suppuration, furunculosis and other manifestations of skin bacterial infection.

Use the thermostable composition with the activity of interferon gamma and lysostaphin 500.000 IU / g and 50 U / g, respectively, as described in Example 1 to prepare the product.

Use 20 g of carboxymethylcellulose, 10 g of sodium alginate and 0.05 g of sodium benzoate to prepare a filling substrate. Use water in amount 100 ml as a solvent. Add the thermostable composition in the amount of 10 mg per 1 g of gel. Prepare the solution at room temperature, mix it with periodic stirring for 30 minutes until the filling substrate is completely swollen. Package the prepared gel into 10-, 25- and 50-g jars.

In the obtained product the activity of recombinant interferon gamma human and lysostaphin is 5.000 IU / g and 0.5 U / g, respectively.

Application instruction: use a dispenser to squeeze out the product on a spatula or cotton tampon and distribute in evenly on the problematic areas of skin. Use as needed, but at least 2 times a day. Duration of the course is from 2 to 3 weeks.

Shelf life of the gel is 3 years.

### EXAMPLE 40. A medicinal product for treatment of skin infections in the form of spray.

Dissolve 200 g trehalose, 4 g potassium sorbate, 30 g PEG400 in 5 1 of water. Stir for at least 10 minutes. pH should be 6.0. Use triethanolamine to adjust pH. Dissolve 2.5 g of the thermostable composition prepared as described in Example 2 in a small volume of water and introduce the solution into the reactor. Avoid foaming. Then adjust the volume to 10.0 1 with water. After 10 minutes, turn off the reactor stirrer.

After sterilizing filtration, pour the product into vials supplied with a spraying device.

Use the product by aerosol spraying on the skin surface.

Shelf life of the product is at least 3 years when stored at room temperature.

The product in the form of spray is used to treat inflammation, suppuration, furunculosis and other manifestations of skin bacterial infection.

Use as needed, but at least 2 times a day. Duration of the course is from 2 to 3 weeks.

In the obtained product the activity of recombinant interferon gamma human and lysostaphin is 5.000 IU / g and 0.5 U / g, respectively.

Use the components in the following ratio per 1 ml of the product:
thermostable composition 2.5 mg
trehalose 20 mg
triethanolamineto provide pH 6.0
potassium sorbate 0.4 mg
PEG4003mg
waterup to 1 ml

### EXAMPLE 41. A lyophilized product for preparation of intranasal solution.

Pour 500 ml of water into a container with a stirring device. Add sodium hydrophosphate dodecahydrate in the amount of 2.19 g, sodium dihydrophosphate dihydrate in the amount of 0.6 g, polyglukin in the amount of 12 g, trehalose in the amount of 1 g. Mix and measure pH, which should be 7.0. Add 10 g of the thermostable composition prepared as described in Example 2 into the container with dissolved ingredients and mix.

Use water to adjust the solution to 1 l and stir for 15 minutes. Avoid foaming. Filter the prepared solution through the filters with a pore size of 0.45 µm and 0.2 µm and aseptically pour into neutral glass vials with a filling dose of 1 ml. Freeze the mixture at minus 40 ° C and dry for 36 hours. Seal the vials with rubber stoppers and aluminum caps.

The product represents a lyophilized hygroscopic white powder. pH of one gram of the product dissolved in 5 ml of water should be 7.0.

In the obtained product the activity of recombinant interferon gamma human and lysostaphin is 20.000 IU / g and 2 U / g, respectively.

The product contains components in the following ratio:

| | |
|---|---|
| Thermostable composition | 10 mg |
| sodium hydrophosphate dodecahydrate | 2.19 mg |
| sodium dihydrophosphate dihydrate | 0.6 mg |
| polyglucin | 12mg |
| trehalose | 1mg. |

The lyophilized product can be stored at a temperature of + 25 ° C for 5 years. Control the stability of the product during storage. After 5 years, lysostaphin activity should be 92% of the initial.

### EXAMPLE 42. A medicinal product in the form of intranasal spray.

Pour 500 ml of water into a container with a stirring device. Add 1.35 g of sodium hydrophosphate dodecahydrate, 0.98 g of sodium dihydrophosphate dihydrate, 30 g of PEG 400, 30 mg of sodium chloride, 1.5 g of sorbitol, 0.3 g of sodium benzoate, 20 g of glycerol. Stir the mixture and measure pH, which should be 6.6. Dissolve 50 g of the thermostable composition prepared as described in Example 2 in a small volume of water, add the solution into the container with dissolved ingredients and mix.

Avoid foaming. After 5 minutes, adjust the volume to 1.0 L with water. Stir for 5 minutes.

After sterilizing filtration, package the product is packaged in 5- and 50-ml vials with a spraying device.

The product represents a colorless liquid. Light opalescence is possible.

In the obtained product the activity of recombinant interferon gamma human and lysostaphin is 100.000 IU / g and 10 U / g, respectively.

The product is not toxic and does not cause side effects.

Use 3-5 times as needed, but at least 3 times a day.

1 ml of the product contains the components in the following ratio:

| | |
|---|---|
| thermostable composition | 50 mg |
| sodium chloride | 0.3mg |
| sorbitol | 1.5mg |
| sodium benzoate | 0.3mg |
| PEG400 | 30 mg |
| glycerol | 20 mg |
| water up to 1 ml. | |

Shelf life of the product is at least 3 years when stored at room temperature.

### EXAMPLE 43. A medicinal product in the form of suppository.

Place 3 g of Tween 80, 15.5 g of polyethylene oxide 400 into a homogenizer and mixed until completely homogenized, then add 0.01 g of ethylenediaminetetraacetic disodium salt and mix until completely dissolved. Then add 0.5 g of zinc sulfate monohydrate and homogenize thoroughly. Add 1 g of the thermostable composition prepared as described in Example 2 in small portions to the homogenate and heat to 48-50 ° C. Pour 0.2 ml of purified water to the obtained homogeneous suspension under stirring. Mix the homogenate thoroughly and pour gradually into 130 g of melted polyethylene oxide 1500 under thorough mixing. Pour the obtained product quickly into a contour cell blister pack No. 5. 100 suppositories with a weight of 1.5 g are obtained, each containing interferon gamma and lysostaphin of the activity of 20.000 IU and 2 U, respectively.

### EXAMPLE 44. A medicinal product in the form of suppository.

Mix a stabilizer butyloxytoluene with dimethylsulfoxide and Tween-80 to prepare 150 g of the mixture. Combine the homogenate with the mixture of melted confectionery fat and paraffin in a ratio of 8: 1 (lipophilic base) and add 0.5 g of the thermostable composition prepared as described in Example 3. Pour the mixture is into suppository molds, cool it and package.

The components are taken in the following ratio (per 1 g of the mixtue):
thermostable composition3.3 mg
butylhydroxytoluene 0.0006 g
   dimethyl sulfoxide 0.5 g
TWIN-80 0.004 g
substrate q.s.

Prepare 100 suppositories with a weight of 1.5 g, each containing interferon gamma and lysostaphin with the activity of 5000 IU and 0.5 U, respectively.

## Claims

1. A thermostable composition possessing antiviral and antibacterial activity represents a lyophilized product containg the following components in 1 g of the comosition:
human recombinant interferon gamma
with activity of 500000 to 2000000IU/g0.03 - 0.12 mg
lysostaphin with activity of 50 to 200 U/g 0.17 - 0.68 mg
methoxy polyethylene glycol 30 - 150 mg
sodium alginate 10 - 25 mg
sodium dihydrophosphate dihydrate 120 - 250 mg
citric acid 35 - 110 mg
dextran q.s.

2. A thermostable composition according to claim 1 **characterized in that** the lyophilized product is obtained from a solution with pH 5-7.

3. A thermostable composition according to claim 1 **characterized in that** the recombinant gamma interferon human is obtained from E. coli producer strain transformed with plasmid pGIF315.

4. A thermostable composition according to claim 1 **characterized in that** dextran with a molecular weight of 60.000 or 70.000 or a mixture of dextran with these molecular weights is used.

5. A thermostable composition according to claim 1 **characterized in that** methoxypolyethylene glycol having a molecular weight of 2000 to 5000 or a mixture of methoxypolyethylene glycol with these molecular weights is used.

6. A thermostable composition according to claim 1 **characterized in that** recombinant lysostaphin is used as lysostaphin.

7. An oral cavity care product representing an aqueous solution of the thermostable composition according to any one of claims 1-6.

8. An oral cavity care product according to claim 7 **characterized in that** the aqueous solution has the activity of recombinant interferon gamma human from 200 to 1000 IU / ml and that of lysostaphin from 0.02 to 0.1 U / ml.

9. An oral cavity care product according to claim 8 **characterized in that** it additionally contains sorbitol, sodium chloride and a preservative.

10. An oral cavity care product according to claim 8 **characterized in that** it additionally contains povidone, potassium sorbate, and / or potassium lactate, flavoring agent and a preservative.

11. An oral cavity care product according to claim 10 **characterized in that** it represents a spray.

12. A toothpaste comprises the thermostable composition according to any of claims 1- 6 and suitable fillers used to prepare toothpastes, and the toothpaste has the activity of human recombinant interferon gamma from 200 to 1000 IU / g and that of lysostaphin from 0.02 to 0.1 ED / g, with the following ratio of components (mass%):
thermostable composition 0.01 - 0.2 % wt.
suitable fillerq.s.

13. A toothpaste according to claim 12 **characterized in that** it additionally comprises vitamins and / or antifungal agents or a mixture them.

14. A sanitary-hygienic product contains a fabric base and the thermostable composition according to any of claims 1-6, and the product has the activity of human recombinant interferon gamma from 5000 to 20,000 IU / cm² and that of lysostaphin from 0.5 to 2 U / cm² with the following ratio of components (mass%):
thermostable composition 0.25 - 4
fabric base q.s.

15. A sanitary-hygienic product according to claim 14 **characterized in that** it additionally contains carboxymethylcellulose, sodium alginate, trehalose, a preservative, which before mixing with the thermostable composition according to any of items from 1 to 6 are dissolved in water and the fabric base is impregnated with the prepared aqueous solution and then dried to a moisture content of 1-7 wt% with the following ratio of components in the end product (wt%):
thermostable composition 0.25 - 4
carboxymethyl cellulose 5 - 40
trehalose 0.05-0.5
sodium alginate 1 - 5
preservative 0.1 - 0.5
fabric base q.s.

16. A sanitary-hygienic product according to claim 14 **characterized in that** the thermostable composition according to any of claims 1-6 and is applied to a fabric base.

17. A sanitary-hygienic product according to claim 14 **characterized in that** the fabric base consists of cotton and / or cellulose and / or viscose and / or synthetic materials or a mixture of them.

18. A sanitary-hygienic product according to any of claims from 14 to 17 **characterized in that** it is preferably represents a wipe, face mask, paper towel and handkerchiefs.

19. A sanitary-hygienic product according to claim 18 **characterized in that** it is packaged in an individual sealed pack or in a batch of hermetic packs.

20. A sanitary-hygienic wet product comprising a fabric base impregnated with the thermostable composition according to any of claims 1-6 dissolved in an aqueous solution containing carboxymethyl cellulose, sodium alginate and a preservative and dried to a moisture content of 10 to 20% by weight, the product has the activity of human recombinant interferon gamma 4000-16000 IU / cm² and that of lysostaphin 0.4 - 1.6 U / cm² with and the following ratio of components in the end product (mass%):
thermostable composition 0.2 - 3.2
carboxymethylcellulose 10 - 25
sodium alginate 2 - 10
preservative 0.05 - 0.15
absorbent substrate q.s.

21. A sanitary-hygienic wet product according to claim 20 **characterized in that** it additionally contains glycerin and / or thioglycol and / or antifungal agents and / or vitamins and / or dye and / or flavoring or a mixture them, and the absorbent substrate is composed of cotton, and / or viscose, and / or synthetic materials or a mixture them.

22. A sanitary-hygienic wet product according to any of claims 20-21 **characterized in that** it is preferably represents a wipe, wet toilet paper, sanitary wipe, and the product is packaged in an individual sealed pack or in a batch of hermetic packs.

23. A disposable absorbent product containing a substrate and a sanitary hygienic product according to any of claims 14-19, and a weight-to-weight ratio basic substrate: product being (5-10): (1-5).

24. A disposable absorbent product according to claim 23 **characterized in that** the absorbent product represents preferably a tampon, hygienic liner, daily hygienic liner.

25. A disposable absorbent product according to claim 24 **characterized in that** the product is packaged into an individual sealed pack or in a batch of sealed packs.

26. A skin care product containing a cosmetically suitable base and the thermostable composition according to any of claims 1-6, and the product has the activity of human recombinant interferon gamma from 500 to 2000 IU / g and that of lysostaphin from 0.05 to 0.2 U / g with the following ratio of the components in the end product (mass%):
thermostable composition 0.025-0.4
cosmetically suitable base q.s.

27. A skin care product according to claim 26 **characterized in that** the cosmetically suitable base represents a gel or a fat base, or an aqueous base, or a powder base and the product represents a cream, gel, jelly, powder, lotion, spray or a mask.

28. A skin care product according to claim 27 **characterized in that** it contains talc and / or aerosil as a powder base.

29. A skin care product according to claim 26 **characterized in that** it additionally contains spermaceti and / or vitamins and / or mineral salts and / or antifungal agents or a mixture of them.

30. A skin care product according to any of claims 26-29 **characterized in that** it represents a product for veterinary use.

31. A sanitary-hygienic product containing an acceptable liquid base and the thermostable composition according to any of claims 1-6, and the product has the activity of human recombinant interferon gamma from 500 to 100,000 IU / ml and that of lysostaphin from 0.05 to 10 U / ml with the following ratio of the components in the end product (mass%):
thermostable composition 0.025 - 20
acceptable liquid base q.s.

32. A sanitary-hygienic product according to claim 31 **characterized in that** it represents a spray and additionally contains trehalose, a preservative and PEG 400 and / or triethanolamine to provide pH 6.0-8.0.

33. A sanitary-hygienic product according to claim 31 **characterized in that** it is prepared in the form of gel and additionally contains carboxymethylcellulose, sodium alginate, a preservative, trehalose and PEG 400 and / or triethanolamine to provide pH 6.0 - 8.0.

34. A sanitary-hygienic product according to any of claims 31-33 **characterized in that** it is the product for surface treatment.

35. A sanitary-hygienic product according to claim 32 **characterized in that** it represents a product for clothes treatment.

36. A laundry conditioner comprises an acceptable liquid base and the thermostable composition according to any of claims 1-6, at the same time the conditioner has the activity of recombinant interferon gamma human 2000-10,000 IU / ml and that of lysostaphin 0.2-1 U / mL with the following ratio of components in the end product (mass%):
thermostable composition 0.1 - 2
acceptable liquid base q.s.

37. A condom lubricant contains the thermostable composition according to any of claims 1-6 and pharmaceutically acceptable additives, at the same time the product has the activity of recombinant interferon gamma human from 5 to 50 U / g and that of lysostaphin from 0.0005 to 0.005 U/ g with the following ratio of components in the end product (mass%):
thermostable composition 0.00025 - 0.01
pharmaceutically acceptable target additives q.s.

38. A condom lubricant according to claim 37 **characterized in that** it additionally contains vitamins and / or antifungal agents.

39. A medicinal product comprising a pharmaceutically acceptable base and the thermostable composition according to any of claims 1-6, and the product has the activity of recombinant interferon gamma human from 5000 to 100,000 IU / g and that of lysostaphin from 0.5 to 10 U / g, with the following ratio of components in the end product (mass%):
thermostable composition 0.25 - 20
pharmaceutically acceptable base q.s.

40. A medicinal product according to claim 39 **characterized in that** it represents either a suppository, spray, ointment, gel or a lyophilized powder.

41. A medicinal product comprises a fabric base and the thermostable composition according to any of claims 1-6; the product has the activity of recombinant interferon gamma human 5000-2000 IU / cm² and that of lysostaphin 0.5-2 U / cm² and the following ratio of components (wt%):
thermostable composition 0.25 - 4
fabric base q.s.

42. A medicinal product according to claim 41 **characterized in that** it additionally contains carboxymethylcellulose, sodium alginate, trehalose and a preservative, which prior to mixing with the thermostable composition according to any of items from 1 to 6 are dissolved in water, and the fabric base is impregnated with the obtained aqueous solution and then dried to a moisture content from 1 to 7% by weight with the following ratio of components in the end product (wt%):
thermostable composition 0.25 - 4
carboxymethyl cellulose 5 - 40
trehalose 0.05-0.5
sodium alginate 1 - 5
preservative 0.1 - 0.5
fabric base q.s.

43. A medicinal product according to claim 41 **characterized in that** the thermostable composition according to any of claims 1-6 is applied to the fabric base.

44. A medicinal product according to claim 41 **characterized in that** the fabric base is made of cotton and / or cellulose and / or viscose and / or synthetic materials or a mixture them, and the product is preferably used as a face mask, bandage, disposable gloves or a sponge for topical use.

45. A medicinal product according to claim 44 **characterized in that** it is packaged in individual sealed packs or in a batch of sealed packs.
